# EUROPEAN PATENT SPECIFICATION

(11) **EP 2 062 574 B1**
(45) Date of publication and mention of the grant of the patent: **25.03.2015**
(21) Application number: 07806928.3
(22) Date of filing: 07.09.2007
(51) Int. Cl.: A61K 31/122, A23K 1/16, A23L 1/30, A61K 9/20, A61K 9/48, A61K 47/10, A61K 47/14, A61K 47/24, A61K 47/26, A61K 47/44

(54) **COMPOSITION COMPRISING REDUCED COENZYME Q10 AND LYSOLECITHIN**
ZUSAMMENSETZUNG MIT REDUZIERTEM COENZYM Q10 UND LYSOLECITHIN
COMPOSITION COMPRENANT UNE COENZYME Q10 RÉDUITE ET DE LA LYSOLÉCITHINE

(30) Priority: 08.09.2006 JP 2006243622; 18.09.2006 US 845255 P; 28.12.2006 US 882466 P
(43) Date of publication of application: 27.05.2009
(73) Proprietor: KANEKA CORPORATION, Osaka (JP)
(72) Inventor: UEDA, Takahiro, Kobe-shi, Hyogo 655-0872 (JP); KITAMURA, Shiro, Akashi-shi, Hyogo 673-0882 (JP); SHINAGAWA, Yoshiyuki, Akashi-shi, Hyogo 674-0051 (JP); KAWABE, Taizou, Himeji-shi, Hyogo 672-8044 (JP); KISHIDA, Hideyuki, Kakogawa-shi, Hyogo 675-0039 (JP); HOSOE, Kazunori, Takasago-shi, Hyogo 676-0025 (JP)
(74) Representative: Hart-Davis, Jason
(86) International application number: PCT/JP2007/067487
(87) International publication number: WO 2008/029909

(56) References cited:
- EP-A1- 1 475 363
- EP-A1- 1 475 363
- EP-A1- 2 039 353
- WO-A1-96/36352
- WO-A1-99/47001
- WO-A1-03/032967
- WO-A1-2006/035900
- JP-A- 10 109 933
- JP-A- 2003 026 625
- JP-A- 2003 119 126
- JP-A- 2003 300 870
- JP-A- 2006 111 596
- DATABASE CAPLUS [Online] 1992 SWENSON E. ET AL.: 'Intestinal permeability enhancement for proteins, peptides and other polar drugs : mechanisms and potential toxicity', XP003021675 Database accession no. (1992:91190) & ADVANCED DRUG DELIVERY REVIEWS vol. 8, no. 1, pages 39 - 92
- DATABASE CAPLUS [Online] 2004 LIU H. ET AL.: 'Effects of five absorption enhancers on intestinal absorption of insulin', XP003021676 Database accession no. (2005:528963) & ZHONGGUO YAOXUE ZAZHI (BEIJING, CHINA) vol. 39, no. 4, pages 276 - 278
- DATABASE CAPLUS [Online] 1977 SKLAN D. ET AL.: 'The effect of lipids on taurocholate absorption from intestinal loops in the rat', XP003021677 Database accession no. (1977:118254) & LIPIDS vol. 12, no. 2, pages 193 - 197

## Description

The present invention relates to a composition having an increased absorbability of reduced coenzyme Q₁₀. Reduced coenzyme Q₁₀ is a compound that exhibits higher oral absorbability compared to oxidized coenzyme Q₁₀, and that is useful as a food, a food with nutrient function claims, a food for specified health uses, a nutritional supplement, a nutritional product, an animal drug, a beverage, a feed, a pet food, a cosmetic product, a pharmaceutical, a therapeutic drug, a prophylactic drug and the like.

### Background Art

Being a benzoquinone derivative known to be widely distributed in the biological kingdom, oxidized coenzyme Q₁₀ is also called vitamin Q because of its vitamin-like function, and is an ingredient that revitalizes the body as a nutrient product for restoring a healthy state from weakened cell activity. On the other hand, reduced coenzyme Q₁₀ is the 2-electron reductant of oxidized coenzyme Q₁₀; whereas oxidized coenzyme Q₁₀ occurs as an orange crystal, reduced coenzyme Q₁₀ occurs as a white crystal. Reduced coenzyme Q₁₀ and oxidized coenzyme Q₁₀ are localized in mitochondria, lysosome, Golgi's apparatus, microsome, peroxisome, or cell membrane and the like, and are known as substances that are essential for the maintenance of the functions of living organisms, involved in ATP production potentiation, antioxidant action in vivo, and membrane stabilization as constituents of the electron transfer system.

Against this background, oxidized coenzyme Q₁₀ has been produced by commonly known conventional methods such as synthesis, fermentation, and extraction from natural substances, and has been used as a pharmaceutical and a health food. On the other hand, reduced coenzyme Q₁₀ is also known to be obtained by producing coenzyme Q₁₀ by commonly known conventional methods such as synthesis, fermentation, and extraction from natural substances, and then concentrating the reduced coenzyme Q₁₀ fraction in the effluent by chromatography and the like (patent reference 1). However, the reduced coenzyme Q₁₀ thus obtained cannot always be in a highly pure state; for example, it is likely to be obtained as a low-purity crystal, oily substance or semi-solid containing impurities such as oxidized coenzyme Q₁₀.

The present inventors diligently investigated and, as a result, established a process for obtaining reduced coenzyme Q₁₀ of high quality and a method of stably storing reduced coenzyme Q₁₀, for which patent applications have been filed (for example, patent references 2 to 5).

Additionally, because coenzyme Q₁₀ is an oil-soluble substance and is hence practically insoluble in water, it is sometimes poorly absorbable from the gastrointestinal tract and often fails to exhibit its efficacy, though it is a useful substance as described above. In these circumstances, various methods have been investigated for increasing the absorbability of oxidized coenzyme Q₁₀. However, because almost no supplies of reduced coenzyme Q₁₀ are commercially available, little research into a method for increasing the absorbability of reduced coenzyme Q₁₀ and a composition therefor has been conducted to date, except for a report that the absorbability of reduced coenzyme Q₁₀ can be increased by preparing a composition comprising reduced coenzyme Q₁₀ wherein a polyglycerol fatty acid ester is co-present as a surfactant (patent reference 5).

Generally, some methods have conventionally been attempted to improve the absorbability of oil-soluble substances; for example, a synthetic surfactant such as a polyoxyethylene sorbitan fatty acid ester or a sorbitan fatty acid ester is allowed to be co-present and the like. However, these synthetic surfactants are often required in large amounts to obtain expected effects, and in preparing a preparation comprising an oil-soluble active ingredient, it is sometimes impossible to add the desired amount. Additionally, large amounts of synthetic surfactants are known to damage the skin and mucosa in some cases, and their toxicity, irritancy and the like are problematic. Furthermore, although what is called a common lecithin (phospholipid) derived from soybean or egg yolk is sometimes used as a natural surfactant to improve the absorbability of an oil-soluble substance, the effect thereof is often insufficient because of the choice of oil-soluble substance, and the results are not always satisfactory. Because reduced coenzyme Q₁₀ has a property of being highly oxidizable in air, the results obtained could be unsatisfactory in terms of oxidation stability if these methods of improving the absorbability of other common oil-soluble substances are simply applied.
patent reference 1: JP 10109933
patent reference 2: WO03/006408
patent reference 3: WO03/006409
patent reference 4: WO03/032967
patent reference 5: WO03/062182

### Disclosure of the Invention

### Problems to Be Solved by the Invention

In view of the above-described circumstances, it is an object of the present invention to provide a composition comprising reduced coenzyme Q₁₀ as a composition capable of increasing the absorbability of reduced coenzyme Q₁₀, compared to conventionally known compositions, when used as a food, a food with nutrient function claims, a food for specified health uses, a nutritional supplement, a nutritional product, an animal drug, a beverage, a feed, a pet food, a cosmetic, a pharmaceutical, a therapeutic drug, or a prophylactic drug and the like.

### Means of Solving the Problems

The present inventors diligently investigated to solve the above-described problems and, as a result, found that the absorbability of reduced coenzyme Q₁₀ can be improved by preparing a composition wherein lysolecithin and reduced coenzyme Q₁₀ are co-present, rather than what is called a common lecithin (phospholipid).

Accordingly, the present invention relates to the following:
1. A composition comprising reduced coenzyme Q₁₀ and lysolecithin wherein the weight ratio of reduced coenzyme Q₁₀ and lysolecithin is not less than about 10/90 and not more than about 90/10.
2. The composition of claim 1, wherein the content of reduced coenzyme Q₁₀ in the composition is not less than 0.1 wt% of the total weight of the composition.
3. The composition of claim 1 or 2, wherein the content of lysolecithin in the composition is not less than 0.1 wt% of the total weight of the composition.
4. The composition of any one of claims 1 to 3, further comprising at least one kind selected from the group consisting of an oil and fat, a surfactant, a higher fatty acid, ethanol and water.
5. The composition of claim 4, wherein the oil and fat is at least one kind selected from the group consisting of safflower oil, olive oil, almond oil, rice oil, rapeseed oil and cottonseed oil.
6. The composition of claim 4, wherein the oil and fat is an oil and fat wherein oleic acid accounts for not less than 50% of the constituent fatty acids thereof.
7. The composition of claim 4, wherein the surfactant is at least one kind of a glycerol fatty acid ester and an organic acid monoglyceride.
8. The composition of claim 7, wherein the glycerol fatty acid ester is at least one kind of surfactant selected from the group consisting of triglycerol monostearate, pentaglycerol trimyristate, decaglycerol monooleate, decaglycerol monostearate, tetraglycerol monolaurate, hexaglycerol monooleate, monoglycerol caprylate, monoglycerol linolate, and monoglycerol stearate.
9. The composition of claim 4, wherein the higher fatty acid is oleic acid.
10. The composition of any one of claims 4 to 9, wherein the composition is liquid or slurry.
11. The composition of claim 10, wherein the total amount of oil and fat, surfactant, higher fatty acid, ethanol and water to the total weight of the composition is not less than 20 wt%.
12. A tablet, powder, chewable tablet, pill, or capsule obtained by processing the composition of any one of claims 1 to 11.
13. The capsule of claim 12, which is a soft capsule.
14. A method of increasing the absorbability of reduced coenzyme Q₁₀ comprising preparing a composition wherein reduced coenzyme Q₁₀ is ingested in the co-presence of lysolecithin.

### Effect of the Invention

According to the present invention, a composition comprising reduced coenzyme Q₁₀ with improved absorbability can be provided by simply preparing a composition containing lysolecithin and reduced coenzyme Q₁₀ in combination, without adding plural components.

### Best Mode for Embodying the Invention

The present invention is hereinafter described in detail. Herein, when coenzyme Q₁₀ is simply referred to as is, it does not matter whether it is of the oxidation form or the reduction form, and if both are present as a mixture, the entire mixture is indicated.

The composition of the present invention is a composition comprising reduced coenzyme Q₁₀ and lysolecithin. The absorbability of reduced coenzyme Q₁₀ can be increased by preparing a composition wherein reduced coenzyme Q₁₀ and lysolecithin are co-present.

In the present invention, reduced coenzyme Q₁₀ may be reduced coenzyme Q₁₀ alone, and may also be used as a mixture with oxidized coenzyme Q₁₀. When coenzyme Q₁₀ is used as the above-described mixture of oxidized coenzyme Q₁₀ and reduced coenzyme Q₁₀, the ratio of reduced coenzyme Q₁₀ to the total amount of coenzyme Q₁₀ (that is, the sum of reduced coenzyme Q₁₀ and oxidized coenzyme Q₁₀) is not subject to limitation, and is, for example, not less than 20 wt%, normally not less than 40 wt%, preferably not less than 60 wt%, more preferably not less than 80 wt%, still more preferably not less than 90 wt%, most preferably not less than 96 wt%. The upper limit is 100 wt%, is not subject to limitation, and is normally not more than 99.9 wt%.

The reduced coenzyme Q₁₀ used in the present invention can be obtained by, for example, commonly known conventional methods such as synthesis, fermentation, and extraction from natural substances, combined with reducing reactions as required, and the like. Preferably, the reduced coenzyme Q₁₀ used is obtained by reducing an existing oxidized coenzyme Q₁₀ such as high-purity coenzyme Q₁₀, or a mixture of oxidized coenzyme Q₁₀ and reduced coenzyme Q₁₀, using a common reducing agent, for example, sodium hydrosulfite (sodium dithionite), sodium borohydride, an ascorbic acid and the like; more preferably, the reduced coenzyme Q₁₀ used is obtained by reducing an existing oxidized coenzyme Q₁₀ such as high-purity coenzyme Q₁₀, or a mixture of oxidized coenzyme Q₁₀ and reduced coenzyme Q₁₀, using an ascorbic acid.

The lysolecithin used in the present invention may be any one wherein one of the acyl groups of what is called a common lecithin (phospholipid) has been hydrolyzed to a hydroxyl group, and the choice thereof is not subject to limitation. The lysolecithin may also contain non-degraded lecithin. As examples of the lecithin (phospholipid) from which the lysolecithin used in the present invention is derived, egg-yolk lecithin, soybean lecithin, phosphatidylcholine, phosphatidylethanolamine, phosphatidylserine, phosphatidylglycerol, phosphatidic acid, phosphatidylinositolamine, cardiolipin, or a mixture thereof and the like can be mentioned. Preferably, the lysolecithin used is soybean lecithin-derived lysolecithin or a mixture of soybean-derived lysolecithin and another lysolecithin.

In the composition of the present invention, the content of reduced coenzyme Q₁₀ in the composition (weight of reduced coenzyme Q₁₀/total weight of composition) is not subject to limitation, and is normally not less than about 0.1 wt%, preferably not less than about 0.5 wt%, more preferably not less than about 1 wt%, particularly preferably not less than about 2 wt%, still more preferably not less than about 3 wt%, most preferably not less than about 5 wt%. The upper limit is not subject to limitation, and is normally not more than about 99.9 wt%, preferably not more than about 99 wt%, more preferably not more than about 95 wt%, particularly preferably not more than about 90 wt%, still more preferably not more than about 80 wt%.

In the composition of the present invention, the content of lysolecithin in the composition (weight of lysolecithin/total weight of composition) is not subject to limitation, and is normally not less than about 0.1 wt%, preferably not less than about 0.5 wt%, more preferably not less than about 1 wt%, particularly preferably not less than about 2 wt%, still more preferably not less than about 3 wt%, most preferably not less than about 5 wt%. The upper limit is not subject to limitation, and is normally not more than about 99.9 wt%, preferably not more than about 99 wt%, more preferably not more than about 95 wt%, particularly preferably not more than about 90 wt%, still more preferably not more than about 80 wt%.

In the composition of the present invention, the weight ratio of reduced coenzyme Q₁₀ and lysolecithin contained in the composition can be subject to limitation; usually, regarding the weight ratio of reduced coenzyme Q₁₀ and lysolecithin (weight of reduced coenzyme Q₁₀/weight of lysolecithin), the lower limit is normally not less than 1/99, preferably not less than 5/95, more preferably not less than 10/90, still more preferably not less than about 15/85, particularly preferably not less than about 20/80. The upper limit can be subject to limitation, and is, for example, not more than 99/1, preferably not more than 95/5, more preferably not more than 90/10, still more preferably not more than 80/20, particularly preferably not more than 70/30, most preferably not more than 60/40.

The material other than reduced coenzyme Q₁₀ and lysolecithin contained in the composition of the present invention is not subject to limitation; for example, an excipient, a disintegrant, a lubricant, a binder, a pigment, an anticoagulant, an absorption promoter, a solubilizing agent, a stabilizer, a flavoring agent, an oil and fat, a surfactant, a higher fatty acid, ethanol, water, an active ingredient other than reduced coenzyme Q₁₀, an antioxidant and the like can be mentioned. Although only reduced coenzyme Q₁₀ and lysolecithin may be used as ingredients with none of these other ingredients contained, it is preferable that of the above-described substances, one kind or more of any of an oil and fat, a surfactant, a higher fatty acid, ethanol, and water be contained as other ingredients; from the viewpoint of improvement of the absorbability, it is more preferable that at least an oil and fat be contained.

The excipient is not subject to limitation; for example, 10 sucrose, lactose, glucose, starch, dextrin, mannitol, crystalline cellulose, calcium phosphate, calcium sulfate and the like can be mentioned.

The disintegrant is not subject to limitation; for example, starch, agar, calcium citrate, calcium carbonate, sodium hydrogen carbonate, dextrin, crystalline cellulose, carboxymethylcellulose, tragacanth, alginic acid and the like can be mentioned.

The lubricant is not subject to limitation; for example, talc, magnesium stearate, polyethylene glycol, silica, hydrogenated oil and the like can be mentioned.

The binder is not subject to limitation; for example, ethylcellulose, methylcellulose, hydroxypropylmethylcellulose, hydroxypropylcellulose, tragacanth, shellac, gelatin, pullulan, gum arabic, polyvinylpyrrolidone, polyvinyl alcohol, polyacrylic acid, polymethacrylic acid, sorbitol and the like can be mentioned.

The pigment is not subject to limitation; for example, pigments such as titanium oxide, food pigment, red iron oxide pigment, safflower pigment, caramel pigment, gardenia pigment, tar pigment, and chlorophyll can be mentioned.

The anticoagulant is not subject to limitation; for example, stearic acid, talc, light silicic anhydride, hydrated silicon dioxide and the like can be mentioned.

The absorption promoter is not subject to limitation; for example, higher alcohols and the like can be mentioned.

The solubilizing agent is not subject to limitation; for example, organic acids such as fumaric acid, succinic acid, and malic acid and the like can be mentioned.

The stabilizer is not subject to limitation; for example, benzoic acid, sodium benzoate, ethyl para-oxybenzoate, beeswax, hydroxypropylmethylcellulose, methylcellulose and the like can be mentioned. Of these stabilizers, hydroxypropylmethylcellulose is preferable because it also contributes to an improvement of the absorbability.

The flavoring agent is not subject to limitation; for example, orange oil, capsicum oil, mustard oil, garlic oil, caraway oil, clove oil, cinnamon oil, cocoa extract, coffee bean extract, ginger oil, spearmint oil, celery seed oil, thyme oil, onion oil, nutmeg oil, parsley seed oil, peppermint oil, vanilla extract, fennel oil, pennyroyal oil, peppermint oil, eucalyptus oil, lemon oil, rose oil, rosemary oil, almond oil, ajowan oil, anise oil, amyris oil, angelica root oil, ambrette seed oil, estragon oil, origanum oil, orris root oil, olibanum oil, cassia oil, cascarilla oil, cananga oil, chamomile oil, calamus oil, cardamon oil, carrot seed oil, cubeb oil, cumin oil, grapefruit oil, cinnamon leaf oil, cade oil, pepper oil, costus root oil, cognac oil, copaiba oil, coriander oil, perilla ocymoides oil, musk, juniper berry oil, star anise oil, sage oil, savory oil, geranium oil, tangerine oil, dill oil, neroli oil, tolu balsam oil, basil oil, birch oil, patchouli oil, palmarosa oil, pimento oil, petigrain oil, bay leaf oil, bergamot oil, Peru balsam oil, benzoin resin, bois de rose oil, hop oil, boronia absolute, marjoram oil, mandarin oil, myrtle oil, yuzu flavor, lime oil, lavandin oil, lavender oil, rue oil, lemon grass oil, lethionine, lovage oil, laurel leaf oil, wormwood oil and the like can be mentioned.

The oil and fat may be a natural oil and fat of animal or vegetable origin, and may be a synthetic oil and fat or a processed oil and fat. More preferably, the oil and fat is one acceptable for food use or pharmaceutical use. As examples of the vegetable oil and fat, coconut oil, palm oil, palm kernel oil, linseed oil, camellia oil, unmilled rice germ oil, rapeseed oil, rice oil, peanut oil, almond oil, corn oil, wheat germ oil, soybean oil, perila oil, cottonseed oil, sunflower oil (sunflower seed oil), kapok oil, evening primrose oil, shea butter, sal butter, cacao butter, sesame oil, safflower oil, olive oil, avocado oil, poppy oil, burdock seed oil and the like can be mentioned; as examples of the animal oil and fat, lard, milk fat, fish oil, beef tallow and the like can be mentioned; furthermore, oils and fats prepared by processing them by separation, hydrogenation, ester exchange and the like (for example, hydrogenated oil) can also be mentioned. Needless to say, a medium-chain triglyceride (MCT) can also be used. The medium-chain triglyceride is not subject to limitation; for example, a triglyceride wherein each fatty acid has 6 to 12, preferably 8 to 12, carbon atoms, and the like can be mentioned. A partial triglyceride of a fatty acid can also be used. Furthermore, a mixture of these oils and fats may be used.

Of the above-described oils and fats, vegetable oils and fats, synthetic oils and fats, processed oils and fats, and medium-chain triglycerides are preferable because of the ease of handling, odor and the like. Furthermore, it is preferable that one be selected from among them in consideration of the price of the oil and fat, the influence on the stability, solubility and absorbability of reduced coenzyme Q₁₀, and the like. For example, coconut oil, palm oil, palm kernel oil, rapeseed oil, rice oil, almond oil, soybean oil, cottonseed oil, safflower oil, olive oil, sunflower oil, MCT and the like are preferable, and rice oil, almond oil, soybean oil, rapeseed oil, safflower oil, olive oil, cottonseed oil, MCT and the like are more preferable. Particularly, from the viewpoint of absorbability, it is preferable that safflower oil, olive oil, almond oil, rice oil, rapeseed oil, cottonseed oil or a mixed oil and fat thereof be contained as the oil and fat in the composition of the present invention; it is more preferable that safflower oil, olive oil, almond oil, rice oil, cottonseed oil or a mixed oil and fat thereof be contained.

When an oil and fat of high oleic acid content, that is, an oil and fat wherein the oleic acid accounts for not less than about 50%, preferably not less than about 60%, more preferably not less than about 70%, of the constituent fatty acids thereof, is used as the fatty acid residue that constitutes the oil and fat, it tends to be possible to further increase the absorbability of reduced coenzyme Q₁₀. As such an oil and fat, oils and fats containing high oleic acid, such as safflower oil containing high oleic acid and rapeseed oil containing high oleic acid, can be mentioned; in particular, safflower oil containing high oleic acid is more preferably used. An oil and fat wherein the oleic acid content accounts for not less than about 50% of the constituent fatty acids thereof is called an oil and fat containing high oleic acid.

As examples of the surfactant, a glycerol fatty acid ester, a sucrose fatty acid ester, an organic acid monoglyceride, sorbitan fatty acid ester, a polyoxyethylene sorbitan fatty acid ester, a propylene glycol fatty acid ester, a condensed ricinoleic acid polyglyceride, saponin and the like can be mentioned. Although the absorbability of reduced coenzyme Q₁₀ is not significantly increased, a common lecithin (phospholipid) other than lysolecithin can also be used as the surfactant.

The glycerol fatty acid ester is not subject to limitation; any of a monoglycerol fatty acid ester and a polyglycerol fatty acid ester can be used. For example, a glycerol fatty acid ester wherein the degree of polymerization of glycerol is 1 to 12, and each fatty acid residue has 6 to 22 carbon atoms, and the like can be mentioned. The fatty acid residue in the glycerol fatty acid ester is not subject to limitation, whether saturated or unsaturated. The number of fatty acid residues in the glycerol fatty acid ester is not subject to limitation because it varies depending on the degree of polymerization of glycerol and the like. The upper limit is the number of hydroxyl groups present in the glycerol skeleton (that is, degree of polymerization of glycerol + 2). The fatty acid residue in the glycerol fatty acid ester is not subject to limitation; one wherein the fatty acid residue has 8 to 22 carbon atoms is preferably used, and one wherein the fatty acid residue has 8 to 18 carbon atoms is particularly preferably used. As examples of such a fatty acid residue, caprylic acid, capric acid, lauric acid, myristic acid, palmitic acid, stearic acid, isostearic acid, oleic acid, linoleic acid, linolenic acid, behenic acid and the like can be mentioned. If two or more fatty acid residues are present, the individual fatty acid residues may be the same or different, but from the viewpoint of the ease of obtainment and the like, they are preferably the same.

Specific examples of the above-mentioned glycerol fatty acid ester include monoglycerol caprylate, diglycerol monocaprylate, diglycerol dicaprylate, diglycerol tricaprylate, diglycerol tetracaprylate, triglycerol monocaprylate, triglycerol dicaprylate, triglycerol tricaprylate, triglycerol tetracaprylate, triglycerol tricaprylate, triglycerol tetracaprylate, triglycerol pentacaprylate, tetraglycerol monocaprylate, tetraglycerol dicaprylate, tetraglycerol tricaprylate, tetraglycerol tetracaprylate, tetraglycerol pentacaprylate, tetraglycerol hexacaprylate, pentaglycerol monocaprylate, pentaglycerol dicaprylate, pentaglycerol tricaprylate, pentaglycerol tetracaprylate, pentaglycerol pentacaprylate, pentaglycerol hexacaprylate, pentaglycerol heptacaprylate, hexaglycerol monocaprylate, hexaglycerol dicaprylate, hexaglycerol tricaprylate, hexaglycerol tetracaprylate, hexaglycerol pentacaprylate, hexaglycerol hexacaprylate, hexaglycerol heptacaprylate, hexaglycerol octacaprylate, heptaglycerol monocaprylate, heptaglycerol dicaprylate, heptaglycerol tricaprylate, heptaglycerol tetracaprylate, heptaglycerol pentacaprylate, heptaglycerol hexacaprylate, heptaglycerol heptacaprylate, heptaglycerol octacaprylate, heptaglycerol nonacaprylate, octaglycerol monocaprylate, octaglycerol dicaprylate, octaglycerol tricaprylate, octaglycerol tetracaprylate, octaglycerol pentacaprylate, octaglycerol hexacaprylate, octaglycerol heptacaprylate, octaglycerol octacaprylate, octaglycerol nonacaprylate, octaglycerol decacaprylate, nonaglycerol monocaprylate, nonaglycerol dicaprylate, nonaglycerol tricaprylate, nonaglycerol tetracaprylate, nonaglycerol pentacaprylate, nonaglycerol hexacaprylate, nonaglycerol heptacaprylate, nonaglycerol octacaprylate, nonaglycerol nonacaprylate, nonaglycerol decacaprylate, nonaglycerol undecacaprylate, decaglycerol monocaprylate, decaglycerol dicaprylate, decaglycerol tricaprylate, decaglycerol tetracaprylate, decaglycerol pentacaprylate, decaglycerol hexacaprylate, decaglycerol heptacaprylate, decaglycerol octacaprylate, decaglycerol nonacaprylate, decaglycerol decacaprylate, decaglycerol undecacaprylate, decaglycerol dodecacaprylate, monoglycerol caprate, diglycerol monocaprate, diglycerol dicaprate, diglycerol tricaprate, diglycerol tetracaprate, triglycerol monocaprate, triglycerol dicaprate, triglycerol tricaprate, triglycerol tetracaprate, triglycerol tricaprate, triglycerol tetracaprate, triglycerol pentacaprate, tetraglycerol monocaprate, tetraglycerol dicaprate, tetraglycerol tricaprate, tetraglycerol tetracaprate, tetraglycerol pentacaprate, tetraglycerol hexacaprate, pentaglycerol monocaprate, pentaglycerol dicaprate, pentaglycerol tricaprate, pentaglycerol tetracaprate, pentaglycerol pentacaprate, pentaglycerol hexacaprate, pentaglycerol heptacaprate, hexaglycerol monocaprate, hexaglycerol dicaprate, hexaglycerol tricaprate, hexaglycerol tetracaprate, hexaglycerol pentacaprate, hexaglycerol hexacaprate, hexaglycerol heptacaprate, hexaglycerol octacaprate, heptaglycerol monocaprate, heptaglycerol dicaprate, heptaglycerol tricaprate, heptaglycerol tetracaprate, heptaglycerol pentacaprate, heptaglycerol hexacaprate, heptaglycerol heptacaprate, heptaglycerol octacaprate, heptaglycerol nonacaprate, octaglycerol monocaprate, octaglycerol dicaprate, octaglycerol tricaprate, octaglycerol tetracaprate, octaglycerol pentacaprate, octaglycerol hexacaprate, octaglycerol heptacaprate, octaglycerol octacaprate, octaglycerol nonacaprate, octaglycerol decacaprate, nonaglycerol monocaprate, nonaglycerol dicaprate, nonaglycerol tricaprate, nonaglycerol tetracaprate, nonaglycerol pentacaprate, nonaglycerol hexacaprate, nonaglycerol heptacaprate, nonaglycerol octacaprate, nonaglycerol nonacaprate, nonaglycerol decacaprate, nonaglycerol undecacaprate, decaglycerol monocaprate, decaglycerol dicaprate, decaglycerol tricaprate, decaglycerol tetracaprate, decaglycerol pentacaprate, decaglycerol hexacaprate, decaglycerol heptacaprate, decaglycerol octacaprate, decaglycerol nonacaprate, decaglycerol decacaprate, decaglycerol undecacaprate, decaglycerol dodecacaprate, monoglycerol laurate, diglycerol monolaurate, diglycerol dilaurate, diglycerol trilaurate, diglycerol tetralaurate, triglycerol monolaurate, triglycerol dilaurate, triglycerol trilaurate, triglycerol tetralaurate, triglycerol trilaurate, triglycerol tetralaurate, triglycerol pentalaurate, tetraglycerol monolaurate, tetraglycerol dilaurate, tetraglycerol trilaurate, tetraglycerol tetralaurate, tetraglycerol pentalaurate, tetraglycerol hexalaurate, pentaglycerol monolaurate, pentaglycerol dilaurate, pentaglycerol trilaurate, pentaglycerol tetralaurate, pentaglycerol pentalaurate, pentaglycerol hexalaurate, pentaglycerol heptalaurate, hexaglycerol monolaurate, hexaglycerol dilaurate, hexaglycerol trilaurate, hexaglycerol tetralaurate, hexaglycerol pentalaurate, hexaglycerol hexalaurate, hexaglycerol heptalaurate, hexaglycerol octalaurate, heptaglycerol monolaurate, heptaglycerol dilaurate, heptaglycerol trilaurate, heptaglycerol tetralaurate, heptaglycerol pentalaurate, heptaglycerol hexalaurate, heptaglycerol heptalaurate, heptaglycerol octalaurate, heptaglycerol nonalaurate, octaglycerol monolaurate, octaglycerol dilaurate, octaglycerol trilaurate, octaglycerol tetralaurate, octaglycerol pentalaurate, octaglycerol hexalaurate, octaglycerol heptalaurate, octaglycerol octalaurate, octaglycerol nonalaurate, octaglycerol decalaurate, nonaglycerol monolaurate, nonaglycerol dilaurate, nonaglycerol trilaurate, nonaglycerol tetralaurate, nonaglycerol pentalaurate, nonaglycerol hexalaurate, nonaglycerol heptalaurate, nonaglycerol octalaurate, nonaglycerol nonalaurate, nonaglycerol decalaurate, nonaglycerol undecalaurate, decaglycerol monolaurate, decaglycerol dilaurate, decaglycerol trilaurate, decaglycerol tetralaurate, decaglycerol pentalaurate, decaglycerol hexalaurate, decaglycerol heptalaurate, decaglycerol octalaurate, decaglycerol nonalaurate, decaglycerol decalaurate, decaglycerol undecalaurate, decaglycerol dodecalaurate, monoglycerol myristate, diglycerol monomyristate, diglycerol dimyristate, diglycerol trimyristate, diglycerol tetramyristate, triglycerol monomyristate, triglycerol dimyristate, triglycerol trimyristate, triglycerol tetramyristate, triglycerol trimyristate, triglycerol tetramyristate, triglycerol pentamyristate, tetraglycerol monomyristate, tetraglycerol dimyristate, tetraglycerol trimyristate, tetraglycerol tetramyristate, tetraglycerol pentamyristate, tetraglycerol hexamyristate, pentaglycerol monomyristate, pentaglycerol dimyristate, pentaglycerol trimyristate, pentaglycerol tetramyristate, pentaglycerol pentamyristate, pentaglycerol hexamyristate, pentaglycerol heptamyristate, hexaglycerol monomyristate, hexaglycerol dimyristate, hexaglycerol trimyristate, hexaglycerol tetramyristate, hexaglycerol pentamyristate, hexaglycerol hexamyristate, hexaglycerol heptamyristate, hexaglycerol octamyristate, heptaglycerol monomyristate, heptaglycerol dimyristate, heptaglycerol trimyristate, heptaglycerol tetramyristate, heptaglycerol pentamyristate, heptaglycerol hexamyristate, heptaglycerol heptamyristate, heptaglycerol octamyristate, heptaglycerol nonamyristate, octaglycerol monomyristate, octaglycerol dimyristate, octaglycerol trimyristate, octaglycerol tetramyristate, octaglycerol pentamyristate, octaglycerol hexamyristate, octaglycerol heptamyristate, octaglycerol octamyristate, octaglycerol nonamyristate, octaglycerol decamyristate, nonaglycerol monomyristate, nonaglycerol dimyristate, nonaglycerol trimyristate, nonaglycerol tetramyristate, nonaglycerol pentamyristate, nonaglycerol hexamyristate, nonaglycerol heptamyristate, nonaglycerol octamyristate, nonaglycerol nonamyristate, nonaglycerol decamyristate, nonaglycerol undecamyristate, decaglycerol monomyristate, decaglycerol dimyristate, decaglycerol trimyristate, decaglycerol tetramyristate, decaglycerol pentamyristate, decaglycerol hexamyristate, decaglycerol heptamyristate, decaglycerol octamyristate, decaglycerol nonamyristate, decaglycerol decamyristate, decaglycerol undecamyristate, decaglycerol dodecamyristate, monoglycerol palmitate, diglycerol monopalmitate, diglycerol dipalmitate, diglycerol tripalmitate, diglycerol tetrapalmitate, triglycerol monopalmitate, triglycerol dipalmitate, triglycerol tripalmitate, triglycerol tetrapalmitate, triglycerol tripalmitate, triglycerol tetrapalmitate, triglycerol pentapalmitate, tetraglycerol monopalmitate, tetraglycerol dipalmitate, tetraglycerol tripalmitate, tetraglycerol tetrapalmitate, tetraglycerol pentapalmitate, tetraglycerol hexapalmitate, pentaglycerol monopalmitate, pentaglycerol dipalmitate, pentaglycerol tripalmitate, pentaglycerol tetrapalmitate, pentaglycerol pentapalmitate, pentaglycerol hexapalmitate, pentaglycerol heptapalmitate, hexaglycerol monopalmitate, hexaglycerol dipalmitate, hexaglycerol tripalmitate, hexaglycerol tetrapalmitate, hexaglycerol pentapalmitate, hexaglycerol hexapalmitate, hexaglycerol heptapalmitate, hexaglycerol octapalmitate, heptaglycerol monopalmitate, heptaglycerol dipalmitate, heptaglycerol tripalmitate, heptaglycerol tetrapalmitate, heptaglycerol pentapalmitate, heptaglycerol hexapalmitate, heptaglycerol heptapalmitate, heptaglycerol octapalmitate, heptaglycerol nonapalmitate, octaglycerol monopalmitate, octaglycerol dipalmitate, octaglycerol tripalmitate, octaglycerol tetrapalmitate, octaglycerol pentapalmitate, octaglycerol hexapalmitate, octaglycerol heptapalmitate, octaglycerol octapalmitate, octaglycerol nonapalmitate, octaglycerol decapalmitate, nonaglycerol monopalmitate, nonaglycerol dipalmitate, nonaglycerol tripalmitate, nonaglycerol tetrapalmitate, nonaglycerol pentapalmitate, nonaglycerol hexapalmitate, nonaglycerol heptapalmitate, nonaglycerol octapalmitate, nonaglycerol nonapalmitate, nonaglycerol decapalmitate, nonaglycerol undecapalmitate, decaglycerol monopalmitate, decaglycerol dipalmitate, decaglycerol tripalmitate, decaglycerol tetrapalmitate, decaglycerol pentapalmitate, decaglycerol hexapalmitate, decaglycerol heptapalmitate, decaglycerol octapalmitate, decaglycerol nonapalmitate, decaglycerol decapalmitate, decaglycerol undecapalmitate, decaglycerol dodecapalmitate, monoglycerol stearate, diglycerol monostearate, diglycerol distearate, diglycerol tristearate, diglycerol tetrastearate, triglycerol monostearate, triglycerol distearate, triglycerol tristearate, triglycerol tetrastearate, triglycerol tristearate, triglycerol tetrastearate, triglycerol pentastearate, tetraglycerol monostearate, tetraglycerol distearate, tetraglycerol tristearate, tetraglycerol tetrastearate, tetraglycerol pentastearate, tetraglycerol hexastearate, pentaglycerol monostearate, pentaglycerol distearate, pentaglycerol tristearate, pentaglycerol tetrastearate, pentaglycerol pentastearate, pentaglycerol hexastearate, pentaglycerol heptastearate, hexaglycerol monostearate, hexaglycerol distearate, hexaglycerol tristearate, hexaglycerol tetrastearate, hexaglycerol pentastearate, hexaglycerol hexastearate, hexaglycerol heptastearate, hexaglycerol octastearate, heptaglycerol monostearate, heptaglycerol distearate, heptaglycerol tristearate, heptaglycerol tetrastearate, heptaglycerol pentastearate, heptaglycerol hexastearate, heptaglycerol heptastearate, heptaglycerol octastearate, heptaglycerol nonastearate, octaglycerol monostearate, octaglycerol distearate, octaglycerol tristearate, octaglycerol tetrastearate, octaglycerol pentastearate, octaglycerol hexastearate, octaglycerol heptastearate, octaglycerol octastearate, octaglycerol nonastearate, octaglycerol decastearate, nonaglycerol monostearate, nonaglycerol distearate, nonaglycerol tristearate, nonaglycerol tetrastearate, nonaglycerol pentastearate, nonaglycerol hexastearate, nonaglycerol heptastearate, nonaglycerol octastearate, nonaglycerol nonastearate, nonaglycerol decastearate, nonaglycerol undecastearate, decaglycerol monostearate, decaglycerol distearate, decaglycerol tristearate, decaglycerol tetrastearate, decaglycerol pentastearate, decaglycerol hexastearate, decaglycerol heptastearate, decaglycerol octastearate, decaglycerol nonastearate, decaglycerol decastearate, decaglycerol undecastearate, decaglycerol dodecastearate, monoglycerol oleate, diglycerol monooleate, diglycerol dioleate, diglycerol trioleate, diglycerol tetraoleate, triglycerol monooleate, triglycerol dioleate, triglycerol trioleate, triglycerol tetraoleate, triglycerol trioleate, triglycerol tetraoleate, triglycerol pentaoleate, tetraglycerol monooleate, tetraglycerol dioleate, tetraglycerol trioleate, tetraglycerol tetraoleate, tetraglycerol pentaoleate, tetraglycerol hexaoleate, pentaglycerol monooleate, pentaglycerol dioleate, pentaglycerol trioleate, pentaglycerol tetraoleate, pentaglycerol pentaoleate, pentaglycerol hexaoleate, pentaglycerol heptaoleate, hexaglycerol monooleate, hexaglycerol dioleate, hexaglycerol trioleate, hexaglycerol tetraoleate, hexaglycerol pentaoleate, hexaglycerol hexaoleate, hexaglycerol heptaoleate, hexaglycerol octaoleate, heptaglycerol monooleate, heptaglycerol dioleate, heptaglycerol trioleate, heptaglycerol tetraoleate, heptaglycerol pentaoleate, heptaglycerol hexaoleate, heptaglycerol heptaoleate, heptaglycerol octaoleate, heptaglycerol nonaoleate, octaglycerol monooleate, octaglycerol dioleate, octaglycerol trioleate, octaglycerol tetraoleate, octaglycerol pentaoleate, octaglycerol hexaoleate, octaglycerol heptaoleate, octaglycerol octaoleate, octaglycerol nonaoleate, octaglycerol decaoleate, nonaglycerol monooleate, nonaglycerol dioleate, nonaglycerol trioleate, nonaglycerol tetraoleate, nonaglycerol pentaoleate, nonaglycerol hexaoleate, nonaglycerol heptaoleate, nonaglycerol octaoleate, nonaglycerol nonaoleate, nonaglycerol decaoleate, nonaglycerol undecaoleate, decaglycerol monooleate, decaglycerol dioleate, decaglycerol trioleate, decaglycerol tetraoleate, decaglycerol pentaoleate, decaglycerol hexaoleate, decaglycerol heptaoleate, decaglycerol octaoleate, decaglycerol nonaoleate, decaglycerol decaoleate, decaglycerol undecaoleate, decaglycerol dodecaoleate, monoglycerol linoleate, diglycerol monolinoleate, diglycerol dilinoleate, diglycerol trilinoleate, diglycerol tetralinoleate, triglycerol monolinoleate, triglycerol dilinoleate, triglycerol trilinoleate, triglycerol tetralinoleate, triglycerol trilinoleate, triglycerol tetralinoleate, triglycerol pentalinoleate, tetraglycerol monolinoleate, tetraglycerol dilinoleate, tetraglycerol trilinoleate, tetraglycerol tetralinoleate, tetraglycerol pentalinoleate, tetraglycerol hexalinoleate, pentaglycerol monolinoleate, pentaglycerol dilinoleate, pentaglycerol trilinoleate, pentaglycerol tetralinoleate, pentaglycerol pentalinoleate, pentaglycerol hexalinoleate, pentaglycerol heptalinoleate, hexaglycerol monolinoleate, hexaglycerol dilinoleate, hexaglycerol trilinoleate, hexaglycerol tetralinoleate, hexaglycerol pentalinoleate, hexaglycerol hexalinoleate, hexaglycerol heptalinoleate, hexaglycerol octalinoleate, heptaglycerol monolinoleate, heptaglycerol dilinoleate, heptaglycerol trilinoleate, heptaglycerol tetralinoleate, heptaglycerol pentalinoleate, heptaglycerol hexalinoleate, heptaglycerol heptalinoleate, heptaglycerol octalinoleate, heptaglycerol nonalinoleate, octaglycerol monolinoleate, octaglycerol dilinoleate, octaglycerol trilinoleate, octaglycerol tetralinoleate, octaglycerol pentalinoleate, octaglycerol hexalinoleate, octaglycerol heptalinoleate, octaglycerol octalinoleate, octaglycerol nonalinoleate, octaglycerol decalinoleate, nonaglycerol monolinoleate, nonaglycerol dilinoleate, nonaglycerol trilinoleate, nonaglycerol tetralinoleate, nonaglycerol pentalinoleate, nonaglycerol hexalinoleate, nonaglycerol heptalinoleate, nonaglycerol octalinoleate, nonaglycerol nonalinoleate, nonaglycerol decalinoleate, nonaglycerol undecalinoleate, decaglycerol monolinoleate, decaglycerol dilinoleate, decaglycerol trilinoleate, decaglycerol tetralinoleate, decaglycerol pentalinoleate, decaglycerol hexalinoleate, decaglycerol heptalinoleate, decaglycerol octalinoleate, decaglycerol nonalinoleate, decaglycerol decalinoleate, decaglycerol undecalinoleate, decaglycerol dodecalinoleate, monoglycerol linolenate, diglycerol monolinolenate, diglycerol dilinolenate, diglycerol trilinolenate, diglycerol tetralinolenate, triglycerol monolinolenate, triglycerol dilinolenate, triglycerol trilinolenate, triglycerol tetralinolenate, triglycerol trilinolenate, triglycerol tetralinolenate, triglycerol pentalinolenate, tetraglycerol monolinolenate, tetraglycerol dilinolenate, tetraglycerol trilinolenate, tetraglycerol tetralinolenate, tetraglycerol pentalinolenate, tetraglycerol hexalinolenate, pentaglycerol monolinolenate, pentaglycerol dilinolenate, pentaglycerol trilinolenate, pentaglycerol tetralinolenate, pentaglycerol pentalinolenate, pentaglycerol hexalinolenate, pentaglycerol heptalinolenate, hexaglycerol monolinolenate, hexaglycerol dilinolenate, hexaglycerol trilinolenate, hexaglycerol tetralinolenate, hexaglycerol pentalinolenate, hexaglycerol hexalinolenate, hexaglycerol heptalinolenate, hexaglycerol octalinolenate, heptaglycerol monolinolenate, heptaglycerol dilinolenate, heptaglycerol trilinolenate, heptaglycerol tetralinolenate, heptaglycerol pentalinolenate, heptaglycerol hexalinolenate, heptaglycerol heptalinolenate, heptaglycerol octalinolenate, heptaglycerol nonalinolenate, octaglycerol monolinolenate, octaglycerol dilinolenate, octaglycerol trilinolenate, octaglycerol tetralinolenate, octaglycerol pentalinolenate, octaglycerol hexalinolenate, octaglycerol heptalinolenate, octaglycerol octalinolenate, octaglycerol nonalinolenate, octaglycerol decalinolenate, nonaglycerol monolinolenate, nonaglycerol dilinolenate, nonaglycerol trilinolenate, nonaglycerol tetralinolenate, nonaglycerol pentalinolenate, nonaglycerol hexalinolenate, nonaglycerol heptalinolenate, nonaglycerol octalinolenate, nonaglycerol nonalinolenate, nonaglycerol decalinolenate, nonaglycerol undecalinolenate, decaglycerol monolinolenate, decaglycerol dilinolenate, decaglycerol trilinolenate, decaglycerol tetralinolenate, decaglycerol pentalinolenate, decaglycerol hexalinolenate, decaglycerol heptalinolenate, decaglycerol octalinolenate, decaglycerol nonalinolenate, decaglycerol decalinolenate, decaglycerol undecalinolenate, decaglycerol dodecalinolenate and the like.

Sucrose fatty acid ester is not particularly limited, and as the fatty acid residue of sucrose fatty acid ester, any can be used whether saturated or unsaturated. The fatty acid residue preferably has 8 to 22 carbon atoms, particularly preferably 8 to 18 carbon atoms. As such fatty acid residue, for example, caprylic acid, capric acid, lauric acid, myristic acid, palmitic acid, stearic acid, isostearic acid, oleic acid, linoleic acid, linolenic acid, behenic acid and the like can be mentioned. When two or more fatty acid residues are present, they may be the same or different. In view of easy availability and the like, they are preferably the same.

While organic acid monoglyceride is not particularly limited, for example, acetic acid monoglyceride (acetic acid monoglycerol), citric acid monoglyceride (citric acid monoglycerol), lactic acid monoglyceride, succinic acid monoglyceride, tartaric acid monoglyceride and the like such as diacetyltartaric acid monoglyceride and the like can be mentioned. Here, fatty acid residue constituting organic acid monoglyceride is not particularly limited. For example, caprylic acid, capric acid, lauric acid, myristic acid, palmitic acid, stearic acid, oleic acid, linoleic acid, linolenic acid, behenic acid and the like can be mentioned. Of these, preferred are myristic acid, palmitic acid, stearic acid, oleic acid and the like.

Sorbitan fatty acid ester is not particularly limited, and as the fatty acid residue of sorbitan fatty acid ester, any can be used whether saturated or unsaturated. The fatty acid residue preferably has 8 to 22 carbon atoms, particularly preferably 8 to 18 carbon atoms. As such fatty acid residue, for example, caprylic acid, capric acid, lauric acid, myristic acid, palmitic acid, stearic acid, isostearic acid, oleic acid, linoleic acid, linolenic acid, behenic acid and the like can be mentioned, with particularly preference given to oleic acid. When two or more fatty acid residues are present, they may be the same or different. In view of easy availability and the like, they are preferably the same.

Polyoxyethylene sorbitan fatty acid ester is not particularly limited, and as the fatty acid residue of polyoxyethylene sorbitan fatty acid ester, any can be used whether saturated or unsaturated. The fatty acid residue preferably has 8 to 22 carbon atoms, particularly preferably 8 to 18 carbon atoms. As such fatty acid residue, for example, caprylic acid, capric acid, lauric acid, myristic acid, palmitic acid, stearic acid, isostearic acid, oleic acid, linoleic acid, linolenic acid, behenic acid and the like can be mentioned, with particularly preference given to oleic acid. When two or more fatty acid residues are present, they may be the same or different. In view of easy availability and the like, they are preferably the same.

Propylene glycol fatty acid ester is not particularly limited, and any of propylene glycol fatty acid monoester and propylene glycol fatty acid diester can be preferably used. Any fatty acid residue of propylene glycol fatty acid ester can be used whether saturated or unsaturated. The fatty acid residue preferably has 6 to 22 carbon atoms, more preferably 8 to 18 carbons, particularly preferably 8 to 12 carbon atoms. As such propylene glycol fatty acid ester, for example, propylene glycol monocaprylate, propylene glycol dicaprylate, propylene glycol monocaprate, propylene glycol dicaprate, propylene glycol monolaurate, propylene glycol dilaurate, propylene glycol monomyristate, propylene glycol dimyristate, propylene glycol monopalmitate, propylene glycol dipalmitate, propylene glycol monostearate, propylene glycol distearate, propylene glycol monoisostearate, propylene glycol diisostearate, propylene glycol monooleate, propylene glycol dioleate, propylene glycol monolinoleate, propylene glycol dilinoleate, propylene glycol monolinoleate, propylene glycol dilinoleate and the like can be mentioned.

While condensed ricinolein acid polyglyceride is not particularly limited and any can be used irrespective of the degree of polymerization of glycerol and the like. For example, one having a degree of polymerization of 2 to 10 can be mentioned. Preferably, degree of polymerization of glycerol is not less than 2, more preferably not less than 3. While the upper limit of the degree of polymerization of glycerol is not particularly limited, it is normally not more than 10, preferably not more than 8, more preferably not more than 6. As such condensed ricinolein acid polyglycerides, for example, condensed ricinolein acid diglyceride, condensed ricinolein acid triglyceride, condensed ricinolein acid tetraglyceride, condensed ricinolein acid pentaglycerides, condensed ricinolein acid hexaglyceride, condensed ricinolein acid octaglyceride and the like can be mentioned. Preferably, condensed ricinolein acid tetraglyceride, condensed ricinolein acid hexaglyceride and the like can be mentioned.

Saponin is not particularly limited, and enju saponin, quillaja saponin, purified soybean saponin, yucca saponin and the like can be mentioned.

General lecithin (phospholipid) other than lysolecithin is not particularly limited and, for example, egg-yolk lecithin, purified soybean lecithin, phosphatidylcholine, phosphatidylethanolamine, phosphatidylserine, sphingomyelin, dicetyl phosphoric acid, stearylamine, phosphatidylglycerol, phosphatidic acid, phosphatidylinositolamine, cardiolipin, ceramide phosphoryl ethanolamine, ceramide phosphoryl glycerol, a mixture thereof and the like can be mentioned.

From the aspect of absorbability, of the above-mentioned surfactants, glycerol fatty acid ester and organic acid monoglyceride are preferable. As the glycerol fatty acid ester, monoglycerol fatty acid ester or polyglycerol fatty acid ester having a degree of polymerization of glycerol of not less than 3 is preferable. In the case of polyglycerol fatty acid ester, the degree of polymerization of glycerol is more preferably not less than 4, still more preferably not less than 5, particularly preferably not less than 6. The degree of polymerization of glycerol is preferably not more than 10. As the polyglycerol fatty acid ester, polyglycerol fatty acid monoester, polyglycerol fatty acid diester and polyglycerol fatty acid triester are preferable, and polyglycerol fatty acid monoester is more preferable.

Specifically, as the organic acid monoglyceride, acetic acid monoglyceride, citric acid monoglyceride, tartaric acid monoglyceride and the like can be mentioned.

As the monoglycerol fatty acid ester, monoglycerol caprylate, monoglycerol stearate, monoglycerol oleate, monoglycerol linoleate and the like can be mentioned. As the polyglycerol fatty acid ester, triglycerol monostearate (monostearic acid triglycerol), pentaglycerol trimyristate (trimyristic acid pentaglycerol), decaglycerol monooleate(decaglycerol monooleic acid ester), decaglycerol monostearate (monostearic acid decaglycerol), tetraglycerol monolaurate, hexaglycerol monooleate and the like can be mentioned.

Of these, acetic acid monoglyceride, citric acid monoglyceride, tartaric acid monoglyceride, triglycerol monostearate, tetraglycerol monolaurate, pentaglycerol trimyristate, decaglycerol monooleate, decaglycerol monostearate, monoglycerol caprylate, monoglycerol linoleate, monoglycerol stearate and the like are particularly preferable.

The higher fatty acid is not particularly limited and, for example, caproic acid, caprylic acid, lauric acid, myristic acid, palmitic acid, stearic acid, oleic acid, linoleic acid, linolenic acid, behenic acid and the like can be mentioned. From the aspect of improved absorbability of reduced coenzyme Q₁₀, oleic acid is preferable from among those.

While water is not particularly limited, tap water, distilled water, ion exchange water and the like can be mentioned.

While ethanol is not particularly limited, ethanol obtained by chemical synthesis, ethanol obtained by sugar fermentation and the like can be mentioned. Of these, ethanol obtained by sugar fermentation is preferable.

As the active ingredient other than reduced coenzyme Q₁₀, for example, amino acid, vitamin, mineral, polyphenol, organic acid, saccharides, peptide, protein, carotenoid and the like can be mentioned.

As the antioxidant, for example, ascorbic acids, tocopherols, vitamin A, β-carotene, sodium bisulfite, sodium thiosulfate, sodium pyrosulfite, citric acids and the like can be mentioned. Of these, ascorbic acids and citric acids are particularly preferable because they preferably suppress oxidization of reduced coenzyme Q₁₀. As ascorbic acids and citric acids, fruit juice condensate (extract, powder etc.), such as lemon, orange, grapefruit and the like containing ascorbic acids or citric acids, may be alternatively used.

The above-mentioned substance may have multiple functions. For example, starch may function as an excipient and a disintegrant, and citric acid may function as 3 roles of a solubilizing agent, an active ingredient other than reduced coenzyme Q₁₀, and an antioxidant.

The composition of the present invention may be in the state of a solid or a solution. Here, the solution state may be a melt of reduced coenzyme Q₁₀, or a state where other liquid component is present, and reduced coenzyme Q₁₀ and/or lysolecithin is dissolved in the liquid component. Needless to say, it may be a slurry wherein a part of reduced coenzyme Q₁₀ and/or lysolecithin is precipitated.

When the composition of the present invention is in the state of a solution or a slurry wherein a part of reduced coenzyme Q₁₀ and/or lysolecithin is precipitated, to afford the desired property (flowability, viscosity etc.), at least one kind selected from the group consisting of fat and oil, surfactant, higher fatty acid, water and ethanol is preferably contained as other component mentioned above.

When the composition of the present invention is in the state of a solution, the content of fat and oil, ethanol, water, higher fatty acid and surfactant in the composition is not particularly limited. The total weight thereof relative to the whole weight of the composition is generally not less than about 20 wt%, preferably not less than about 30 wt%, more preferably not less than about 40 wt%. While the upper limit is not particularly limited, it is generally not more than about 99.5 wt%, preferably not more than about 99 wt%, more preferably not more than about 95 wt%, particularly preferably not more than about 90 wt%.

In this case, the content of fat and oil in the composition is not particularly limited and varies depending on the liquid property and the like. It is generally not more than about 99 wt%, preferably not more than about 95 wt%, more preferably not more than about 90 wt%. The lower limit is naturally 0 wt%, generally not less than about 1 wt%, preferably not less than about 5 wt%, more preferably not less than about 10 wt%.

While the content of surfactant in the composition is not particularly limited, it is generally not more than about 90 wt%, preferably not more than about 80 wt%, more preferably not more than about 70 wt%, particularly preferably not more than about 60 wt%. The lower limit is naturally 0 wt%, generally not less than about 1 wt%, preferably not less than about 3 wt%, more preferably not less than about 5 wt%.

When the composition of the present invention is a solid, to afford the desired property (flowability, viscosity etc.), at least one kind selected from the group consisting of excipient, disintegrant, lubricant, binder, anticoagulant, absorption promoter, solubilizing agent and stabilizer is preferably contained as other component mentioned above, in addition to fat and oil, surfactant, higher fatty acid, ethanol and water.

When the composition of the present invention is a solid, the content of excipient, disintegrant, lubricant, binder, anticoagulant, absorption promoter, solubilizing agent, stabilizer, fat and oil, surfactant, ethanol and water in the composition is not particularly limited. The total weight thereof relative to the whole weight of the composition is generally not less than about 1 wt%, preferably not less than about 3 wt%, more preferably not less than about 5 wt%, particularly preferably not less than about 10 wt%. While the upper limit is not particularly limited, it is generally not more than about 99 wt%, preferably not more than about 95 wt%, more preferably not more than about 90 wt%, particularly preferably not more than about 80 wt%.

In this case, the content of fat and oil in the composition is not particularly limited and is generally not more than about 50 wt%, preferably not more than about 40 wt%, more preferably not more than about 30 wt%. The lower limit is naturally 0 wt%, generally not less than about 1 wt%, preferably not less than about 2 wt%, more preferably not less than about 3 wt%.

While the content of surfactant in the composition is not particularly limited, it is generally not more than about 50 wt%, preferably not more than about 40 wt%, more preferably not more than about 30 wt%. The lower limit is naturally 0 wt%, generally not less than about 1 wt%, preferably not less than about 2 wt%, more preferably not less than about 3 wt%.

The composition of the present invention can be used as it is or processed into food, food with nutrient function, food for specified health uses, nutritional supplement, nutritional product, beverage, animal drug, feed, cosmetics, quasi drug, pharmaceutical product, a therapeutic drug, preventive drug and the like and used. The processed form of the composition of the present invention includes, for example, oral administration form such as capsule (microcapsule, hard capsule, soft capsule), tablet, powder, chewable tablet, pill, syrup, beverage and the like, and further, a form such as cream, suppository, toothpaste and the like. Preferable processed forms are capsule, tablet, powder, chewable tablet and pill, particularly preferably capsule, especially, soft capsule.

When a capsule is prepared, the capsule base material is not particularly limited, and gelatin derived from beef bone, beef skin, pig skin, fish skin and the like, as well as other base materials (e.g., production aids including thickening agent such as products derived from seaweed such as carageenan, alginic acid and the like, products derived from plants and seeds such as locust bean gum, guar gum and the like, products derived from plant secretions such as gum arabic and the like, and the like or celluloses usable as food additive) can be used.

When the composition of the present invention is processed into the above-mentioned form, particularly a soft capsule, it is more preferably liquid (including solution as well as suspension and slurry) at ambient temperature or a temperature higher than that.

The composition of the present invention and the above-mentioned capsule may be used after addition to so-called ordinary foods. As the above-mentioned food, for example, dairy products such as milk, milk beverage, cheese, formula milk, ice cream, yogurt and the like, beverages such as juice, lactic acid beverage, tea, coffee and the like, sweets such as chocolate, cookie, biscuit, candy, Japanese confectionery, rice confectionery, cake, pie, pudding and the like, flour products such as bread, noodles and the like, rice products such as risotto, rice and the like, seasoning such as soybean sauce, miso, mayonnaise, dressing and the like, and the like can be mentioned. It is needless to say that the composition may be used in the form of a fish processed product, a agriculture processed product or a animal processed product or other food form.

Note that reduced coenzyme Q₁₀ is easily oxidized in the air. To suppress such oxidization, the composition of the present invention is preferably prepared and/or preserved, for example, under a deoxygenated atmosphere such as inactive gas atmosphere (e.g. a nitrogen atmosphere and the like) and the like. The above-mentioned processing and preservation after processing are also preferably performed under a deoxygenated atmosphere such as the above-mentioned inactive gas atmosphere and the like.

While the amount of ingestion (dose) of reduced coenzyme Q₁₀ for an adult per day in the present invention varies depending on the age, body weight, general health condition, sex, diet, ingestion (administration) time and the like, from the aspect of the intake of a necessary amount, it is preferably not less than 30 mg, more preferably not less than 50 mg, particularly preferably not less than 100 mg. While the upper limit is not particularly limited, in consideration of the cost etc., it is preferably not more than 1200 mg, more preferably not more than 800 mg, particularly preferably not more than 300 mg. Generally, the amount of reduced coenzyme Q₁₀ is preferably within the range of 30 - 1200 mg, more preferably 50 - 800 mg, particularly preferably 100 - 300 mg. The above-mentioned daily dose can be ingested (administered) at once or in several portions. In addition, the period of ingestion (administration) is not particularly limited.

According to the present invention, a composition containing reduced coenzyme Q₁₀ with improved absorbability can be provided by simply preparing a composition containing lysolecithin and reduced coenzyme Q₁₀ in combination, without adding plural components. The composition of the present invention is superior in the absorbability of reduced coenzyme Q₁₀, irrespective of ingestion (administration) time, whether during hungry (fasting) or after eating (full feeding). Generally, liposoluble substance is known to show low absorbability when ingested (administered) during hungry (fasting). The composition of the present invention is particularly effective since it is superior in the absorbability during hungry (fasting) and does not require specific ingestion period.

Unlike surfactants conventionally used for a composition containing reduced coenzyme Q₁₀, such as polyoxyethylene sorbitan fatty acid ester (for example, Tween80), sorbitan fatty acid ester (Span80) and the like, lysolecithin has been found to not impair the oxidization stability of reduced coenzyme Q₁₀. Therefore, in a composition containing reduced coenzyme Q₁₀ and lysolecithin of the present invention, the absorbability of reduced coenzyme Q₁₀ can be enhanced without impairing the oxidization stability of reduced coenzyme Q₁₀. Thus, the present invention provides a method of enhancing the oxidization stability of reduced coenzyme Q₁₀ , which comprises preparing a composition permitting ingestion of reduced coenzyme Q₁₀ in the co-presence of lysolecithin.

The composition of the present invention comprises reduced coenzyme Q₁₀ and lysolecithin in combination, and only requires combining reduced coenzyme Q₁₀ and lysolecithin on administration (ingestion). Accordingly, it may be a single preparation obtained by simultaneously processing reduced coenzyme Q₁₀ and lysolecithin, or a combination of two kinds of preparations of reduced coenzyme Q₁₀ and lysolecithin, which have been separately produced, as long as the reduced coenzyme Q₁₀ and lysolecithin can be combined on administration. The way of administration is not particularly limited and, for example, (1) administration of a composition containing reduced coenzyme Q₁₀ and lysolecithin, i.e., a single preparation, (2) simultaneous administration of two kinds of preparations of reduced coenzyme Q₁₀ and lysolecithin, which have been separately produced, (3) administration of two kinds of preparations of reduced coenzyme Q₁₀ and lysolecithin, which have been separately produced, in a staggered manner (for example, administration in the order of lysolecithin and reduced coenzyme Q₁₀, or in the reverse order) and the like can be mentioned.

According to the present invention, moreover, a method of enhancing the absorbability of reduced coenzyme Q₁₀, which comprises ingesting (administering) reduced coenzyme Q₁₀ in the co-presence of lysolecithin, can be provided. Ingestion (administration) in the co-presence means the above-mentioned (1) - (3) and the like. In the method of enhancing the absorbability of the present invention, ingestion (administration) is possible whether during hungry (fasting) or after eating (full feeding). In other words, the method of enhancing the absorbability of reduced coenzyme Q₁₀ of the present invention can be preferably performed irrespective of the ingestion (administration) time.

### Examples

The present invention is explained in more detail in the following by referring to Production Examples and Examples, which are not to be construed as limitative. The purity of coenzyme Q₁₀, the ratio (weight ratio) of reduced coenzyme Q₁₀/oxidized coenzyme Q₁₀, and concentrations of reduced coenzyme Q₁₀ and oxidized coenzyme Q₁₀ were determined by the following HPLC analysis.

### (HPLC analysis conditions)

column; SYMMETRY C18 (manufactured by Waters) 250 mm (length) 4.6 mm (inner diameter), mobile phase; C₂H₅OH:CH₃OH=4:3 (v:v), detection wavelength; 210 nm, flow rate; 1 ml/min, retention time of reduced coenzyme Q₁₀; 9.1 min, retention time of oxidized coenzyme Q₁₀; 13.3 min.

### (Production Example 1)

To 1000 g of ethanol were added 100 g of oxidized coenzyme Q₁₀ and 60 g of ascorbic acid, and the mixture was stirred at 78°C to perform a reduction reaction. After 30 hr, the mixture was cooled to 50°C and, while maintaining the same temperature, ethanol 330 g and water 70 g were added. The ethanol solution was cooled to 2°C at a cooling rate of 10°C/hr with stirring to give a white slurry. The obtained slurry was filtered under reduced pressure, and the wet crystal was successively washed with cold ethanol, cold water and cold ethanol (temperature of cold solvent used for washing was 2°C). The wet crystal was further dried under reduced pressure (20 - 40°C, 1 - 30 mmHg) to give a white dry crystal (97 g) (yield 97 mol%). All the above operations were performed in a nitrogen atmosphere. The weight ratio of reduced coenzyme Q₁₀/oxidized coenzyme Q₁₀ in the obtained crystal was 99.5/0.5.

### (Example 1)

### <Preparation of test substance administration liquid>

Rapeseed oil and soybean-derived lysolecithin (EMULTOP IP manufactured by Degussa) were mixed at a weight ratio 9:1 with heating to about 50°C. To the mixture of rapeseed oil/lysolecithin (weight ratio 9/1) was added reduced coenzyme Q₁₀ obtained in Production Example 1, and the mixture was dissolved by heating at about 60°C to give an administration solution (reduced coenzyme Q₁₀/lysolecithin weight ratio: 1/3.3) having a reduced coenzyme Q₁₀ concentration of 3 wt%. For comparison, an administration solution was prepared using soybean lecithin (TOPCITIN-UB manufactured by Degussa) instead of lysolecithin and under the same conditions by the same method.

### <Experiment system>

The above-mentioned test substance administration solution was orally administered to 8-week-old male Sprague-Dawley rats (supplier: Japan SLC, Inc.) at a dose of reduced coenzyme Q₁₀ of 90 mg/kg. The blood was taken from each rat at 1, 2, 4, 8 and 24 hr after test substance administration. The obtained blood was centrifuged to plasma. Thereafter, an oxidization treatment of plasma reduced coenzyme Q₁₀ and an extraction treatment of oxidized coenzyme Q₁₀ were performed and plasma coenzyme Q₁₀ concentration was measured as oxidized coenzyme Q₁₀ using HPLC.

### <Results>

The time course changes of plasma coenzyme Q₁₀ concentration after oral administration of the above-mentioned test substance administration solution is shown in Table 1. In addition, the area under plasma coenzyme Q₁₀ concentration-time curve (AUC) from 1 hr to 8 hr after test substance administration, and from 1 hr to 24 hr are shown in Table 2 and Table 3.

A comparison of changes in the plasma coenzyme Q₁₀ concentration after oral administration of a composition containing lysolecithin and reduced coenzyme Q₁₀ of the present invention with those of a composition containing conventional lecithin instead of lysolecithin has revealed higher plasma concentrations and higher AUC of the composition containing lysolecithin. That is, the composition containing reduced coenzyme Q₁₀ and lysolecithin of the present invention has been clarified to be a composition superior in the oral absorbability as compared to conventional compositions.

**(Table 1)**

| time course changes of plasma coenzyme Q₁₀ concentration (µg/mL) | | |
|---|---|---|
| | test substance composition | |
| blood sampling time (hr) | reduced coenzyme Q₁₀ +lecithin | reduced coenzyme Q₁₀ +lysolecithin |
| 1 | 0.77 | 0.50 |
| 2 | 2.11 | 2.61 |
| 4 | 1.76 | 2.29 |
| 8 | 1.26 | 1.58 |
| 24 | 0.94 | 0.51 |

**(Table 2)**

| area under plasma coenzyme Q₁₀ concentration-time curve (AUC) of 1 - 8 hr after administration (µg/mL×hr) | |
|---|---|
| test substance composition | |
| reduced coenzyme Q₁₀ +lecithin | reduced coenzyme Q₁₀ +lysolecithin |
| 11.3 | 14.2 |

| | |
|---|---|
| The data shows an average value of each n=5. | |

**(Table 3)**

| area under plasma coenzyme Q₁₀ concentration-time curve (AUC) of 1 - 24 hr after administration (µg/mL×hr) | |
|---|---|
| test substance composition | |
| reduced coenzyme Q₁₀ +lecithin | reduced coenzyme Q₁₀ +lysolecithin |
| 28.9 | 31.0 |

### (Example 2)

### <Preparation of test substance administration solution>

MCT (medium chain triglyceride) and soybean-derived lysolecithin (EMULTOP IP manufactured by Degussa) were mixed at a weight ratio 8:2 with heating to about 50°C. After mixing, to MCT/lysolecithin (weight ratio 8/2) was added reduced coenzyme Q₁₀ described in Production Example 1, and the mixture was dissolved by heating to about 60°C to give an administration solution (reduced coenzyme Q₁₀/lysolecithin weight ratio 1/6.5)having a reduced coenzyme Q₁₀ concentration of 3 wt% (sample 2-1). Separately, MCT (medium chain triglyceride), hexaglycerol monooleate (SY-Glyster MO-5S manufactured by Sakamoto Yakuhin Kogyo Co., Ltd.) and soybean-derived lysolecithin (EMULTOP IP, Degussa) were mixed under heating at about 50°C to achieve the weight ratio of 8:1:1. After mixing, to MCT/hexaglycerol monooleate/lysolecithin (weight ratio 8/1/1) was added reduced coenzyme Q₁₀ described in Production Example 1, and the mixture was dissolved by heating to about 60°C to give an administration solution (reduced coenzyme Q₁₀/lysolecithin weight ratio 1/3.3) having a reduced coenzyme Q₁₀ concentration of 3 wt% (sample 2-2). For comparison, an administration solution was prepared using soybean lecithin (EMULPUR IP manufactured by Degussa) instead of lysolecithin and under the same conditions as in sample 2-2 by the same method (sample 2-3).

### <Experiment system>

The above-mentioned test substance administration solution was orally administered to 8-week-old male Sprague-Dawley rats (supplier: Japan SLC, Inc.) at a dose of reduced coenzyme Q₁₀ of 90 mg/kg. The blood was taken from each rat at 1, 2, 4, 8 and 24 hr after test substance administration. The obtained blood was centrifuged to plasma. Thereafter, an oxidization treatment of plasma reduced coenzyme Q₁₀ and an extraction treatment of oxidized coenzyme Q₁₀ were performed and plasma coenzyme Q₁₀ concentration was measured as oxidized coenzyme Q₁₀ using HPLC.

### <Results>

The time course changes of plasma coenzyme Q₁₀ concentration after oral administration of the above-mentioned test substance administration solution is shown in Table 4. In addition, the area under plasma coenzyme Q₁₀ concentration-time curve (AUC) from 1 hr to 8 hr after test substance administration, and from 1 hr to 24 hr are shown in Table 5 and Table 6.

A comparison of changes in the plasma coenzyme Q₁₀ concentration after oral administration of a composition containing lysolecithin and reduced coenzyme Q₁₀ of the present invention with those of a composition containing conventional lecithin instead of lysolecithin and using the same surfactant in combination has revealed higher plasma concentrations and higher AUC of the composition containing lysolecithin of the present invention. That is, the composition containing reduced coenzyme Q₁₀ and lysolecithin of the present invention has been clarified to be a composition superior in the oral absorbability. In addition, by comparison of the presence and absence of the combined use of lysolecithin and surfactant (samples 2-1 and 2-2), the combined use of a surfactant enhances the oral absorbability.

**(Table 4)**

| time course changes of plasma coenzyme Q₁₀ concentration (µg/mL) | | | |
|---|---|---|---|
| sample No. | | | |
| blood sampling time (hr) | (2-1) | (2-2) | (2-3) |
| 1 | 0.08 | 0.09 | 0.09 |
| 2 | 0.34 | 0.44 | 0.36 |
| 4 | 0.94 | 1.24 | 0.71 |
| 8 | 0.94 | 1.22 | 0.64 |
| 24 | 0.49 | 0.50 | 0.37 |

**(Table 5)**

| area under plasma coenzyme Q₁₀ concentration-time curve (AUC) of 1 - 8 hr after administration (µg/mL×hr) | |
|---|---|
| sample No. | |
| (2-2) | (2-3) |
| 6.86 | 3.94 |

| | |
|---|---|
| The data shows an average value of each n=5. | |

**(Table 6)**

| area under plasma coenzyme Q₁₀ concentration-time curve (AUC) of 1 - 24 hr after administration (µg/mL×hr) | | |
|---|---|---|
| sample No. | | |
| (2-1) | (2-2) | (2-3) |
| 16.7 | 20.6 | 11.8 |

| | | |
|---|---|---|
| The data shows an average value of each n=5. | | |

### (Example 3)

### <Preparation of test substance administration solution>

MCT and soybean-derived lysolecithin (EMULTOP IP manufactured by Degussa) were mixed at a weight ratio 8:2 with heating to about 50°C. To the mixture of MCT/lysolecithin (weight ratio 8/2) was added reduced coenzyme Q₁₀ obtained in Production Example 1, and the mixture was dissolved by heating to about 60°C to give an administration solution (reduced coenzyme Q₁₀/lysolecithin weight ratio 1/3.3) having a reduced coenzyme Q₁₀ concentration of 3 wt%. For comparison, an administration solution was prepared using decaglycerol monooleate (SY-Glyster MO-7S manufactured by Sakamoto Yakuhin Kogyo Co., Ltd.) instead of lysolecithin and under the same conditions by the same method.

### <Experiment system>

The above-mentioned test substance administration solution was orally administered to 6- to 8-week-old male Sprague-Dawley rats (supplier: Japan SLC, Inc.) at a dose of reduced coenzyme Q₁₀ of 90 mg/kg. The blood was taken from each rat at 1, 2 and 4 hr after test substance administration. The obtained blood was centrifuged to plasma. Thereafter, an oxidization treatment of plasma reduced coenzyme Q₁₀ and an extraction treatment of oxidized coenzyme Q₁₀ were performed and plasma coenzyme Q₁₀ concentration was measured as oxidized coenzyme Q₁₀ using HPLC.

### <Results>

The maximum plasma coenzyme Q₁₀ concentration (Cmax) after oral administration of the above-mentioned test substance administration solution and the time at that time (Tmax) are shown in Table 7.

A comparison of the changes in the plasma coenzyme Q₁₀ concentration after oral administration of the composition containing lysolecithin and reduced coenzyme Q₁₀ of the present invention with those of a composition containing a general synthesis surfactant such as decaglycerol monooleate has revealed higher maximum plasma coenzyme Q₁₀ concentration of the composition containing lysolecithin. That is, the composition containing reduced coenzyme Q₁₀ and lysolecithin of the present invention has been clarified to be a composition superior in the oral absorbability as compared to conventional compositions.

**(Table 7)**

| maximum plasma coenzyme Q₁₀ concentration (Cmax) and the time at that time (Tmax) | | |
|---|---|---|
| | test substance composition | |
| | reduced coenzyme Q₁₀ +lysolecithin | reduced coenzyme Q₁₀ +decaglycerol monooleate |
| Cmax (µg/mL) | 0.94 | 0.53 |
| Tmax (hr) | 4 | 4 |

### (Example 4)

### <Preparation of test substance administration solution>

Safflower oil (safflower oil containing high oleic acid, oleic acid content in constituent fatty acid : 77%), tetraglycerol monolaurate (SY-Glyster ML-310 manufactured by Sakamoto Yakuhin Kogyo Co., Ltd.) and soybean-derived lysolecithin (EMULTOP IP manufactured by Degussa) were mixed at a weight ratio of 7:1:1 with heating to about 50°C. After mixing, to safflower oil/tetraglycerol monolaurate/lysolecithin (weight ratio 7/1/1) was added reduced coenzyme Q₁₀ described in Production Example 1, and the mixture was dissolved by heating to about 60°C to give an administration solution (reduced coenzyme Q₁₀/lysolecithin weight ratio 1/5.5) having a reduced coenzyme Q₁₀ concentration of 2 wt%. For comparison, an administration solution was prepared using soybean lecithin (EMULPUR IP manufactured by Degussa) instead of lysolecithin and under the same conditions and by the same method. Similarly, oleic acid, tetraglycerol monolaurate and soybean-derived lysolecithin (EMULTOP IP manufactured by Degussa) were mixed at a weight ratio of 7:1:1 with heating to about 50°C. Reduced coenzyme Q₁₀ described in Production Example 1 was added, and the mixture was dissolved by heating to about 60°C to also give an administration solution (reduced coenzyme Q₁₀/lysolecithin weight ratio 1/5.5) having a reduced coenzyme Q₁₀ concentration of 2 wt%.

### <Experiment system>

The above-mentioned test substance administration solution was orally administered to 8-week-old male Sprague-Dawley rats (supplier: Japan SLC, Inc.) at a dose of reduced coenzyme Q₁₀ of 30 mg/kg. The blood was taken from each rat at 1, 2, 4, 8 and 24 hr after test substance administration. The obtained blood was centrifuged to plasma. Thereafter, an oxidization treatment of plasma reduced coenzyme Q₁₀ and an extraction treatment of oxidized coenzyme Q₁₀ were performed and plasma coenzyme Q₁₀ concentration was measured as oxidized coenzyme Q₁₀ using HPLC.

### <Results>

The areas under plasma coenzyme Q₁₀ concentration-time curve (AUC) of 1 hr - 8 hr and 1 hr - 24 hr when the above-mentioned test substance administration solution was orally administered are shown in Table 8 and Table 9.

A comparison of changes in the plasma coenzyme Q₁₀ concentration after oral administration of a composition containing lysolecithin and reduced coenzyme Q₁₀ of the present invention with those of a composition containing conventional lecithin instead of lysolecithin and using the same fat and oil has revealed higher AUC of the composition containing lysolecithin. That is, composition containing reduced coenzyme Q₁₀ and lysolecithin of the present invention has also been clarified to be a composition superior in the oral absorbability. The composition containing reduced coenzyme Q₁₀ and lysolecithin of the present invention has been clarified to be a composition superior in the oral absorbability even when oleic acid, which is a higher fatty acid, is used instead of fat and oil.

**(Table 8)**

| area under plasma coenzyme Q₁₀ concentration-time curve (AUC) of 1 - 8 hr after administration (µg/mL×hr) | |
|---|---|
| test substance composition | |
| reduced coenzyme Q₁₀ +lecithin+safflower oil containing high oleic acid | reduced coenzyme Q₁₀ +lysolecithin+safflower oil containing high oleic acid |
| 7.40 | 8.96 |

| | |
|---|---|
| The data shows an average value of each n=5. | |

**(Table 9)**

| area under plasma coenzyme Q₁₀ concentration-time curve (AUC) of 1 - 24 hr after administration (µg/mL×hr) | | |
|---|---|---|
| test substance composition | | |
| reduced coenzyme Q₁₀ +lecithin+safflower oil containing high oleic acid | reduced coenzyme Q₁₀ +lysolecithin +safflower oil containing high oleic acid | reduced coenzyme Q₁₀ +lysolecithin +oleic acid |
| 17.5 | 23.1 | 20.3 |

| | | |
|---|---|---|
| The data shows an average value of each n=5. | | |

### (Example 5)

### <Preparation of test substance administration solution>

Safflower oil (sufflower oil containing high oleic acid, oleic acid content in constituent fatty acid: 77%), various surfactants described in Table 10 (glycerol fatty acid ester or organic acid monoglyceride), soybean-derived lysolecithin (EMULTOP IP manufactured by Degussa) were mixed at a weight ratio 7:1:1 with heating to about 50°C. After mixing, reduced coenzyme Q₁₀ was added to safflower oil/surfactant/lysolecithin (weight ratio 7/1/1), the mixture was dissolved by heating to about 60°C to give an administration solution (reduced coenzyme Q₁₀/lysolecithin weight ratio 1/5.5) having a reduced coenzyme Q₁₀ (described in Production Example 1) concentration of 2 wt%.

**(Table 10)**

| Experim. No. | surfactant species | trade name | manufacturer | HLB of surfactant |
|---|---|---|---|---|
| (4-1) | acetic acid monoglyceride (fatty acid residue:stearic acid) | poem G-508 | RIKEN VITAMIN CO., LTD. | 0.8 |
| (4-2) | citric acid monoglyceride (fatty acid residue:oleic acid) | SUNSOFT No.623M | Taiyo Kagaku Co., Ltd. | 7 |
| (4-3) | diglycerol monooleate | poem DO-100V | RIKEN VITAMIN CO., LTD. | 8 |
| (4-4) | diglycerol monooleate and diglycerol dioleate mixture | SUNSOFT Q-17B | Taiyo Kagaku Co., Ltd. | 6.5 |
| (4-5) | triglycerol monostearate | SUNSOFT A-181C | Taiyo Kagaku Co., Ltd. | 10.0 |
| (4-6) | tetraglycerol monostearate | SY-Glyster MS-3S | Sakamoto Yakuhin Kogyo Co., Ltd. | 8.4 |
| (4-7) | pentaglycerol monooleate | SUNSOFT A-171E | Taiyo Kagaku Co., Ltd. | 13 |
| (4-8) | pentaglycerol trimyristate | SUNSOFT A-143E | Taiyo Kagaku Co., Ltd. | 8.0 |
| (4-9) | decaglycerol monooleate | Ryoto polyglyester O-15D | Mitsubishi-kagaku Foods corporation | 13 |
| (4-10) | decaglycerol monostearate | SUNSOFT Q-18S | Taiyo Kagaku Co., Ltd. | 12.0 |
| (4-11) | tetraglycerol monolaurate | SY-Glyster ML-310 | Sakamoto Yakuhin Kogyo Co., Ltd. | 10.3 |

### <Experiment system>

The above-mentioned test substance administration solution was orally administered to 10-week-old male Sprague-Dawley rats (supplier: Japan SLC, Inc.) at a dose of reduced coenzyme Q₁₀ of 30 mg/kg. The blood was taken from each rat at 1, 2, 4, 8 and 24 hr after test substance administration. The obtained blood was centrifuged to plasma. Thereafter, an oxidization treatment of plasma reduced coenzyme Q₁₀ and an extraction treatment of oxidized coenzyme Q₁₀ were performed and plasma coenzyme Q₁₀ concentration was measured as oxidized coenzyme Q₁₀ using HPLC.

### <Results>

The changes in the plasma coenzyme Q₁₀ concentration and areas under plasma coenzyme Q₁₀ concentration-time curve (AUC) of 1 hr - 24 hr when the above-mentioned test substance administration solution was orally administered are shown in Table 11.

**(Table 11)**

| time course changes of plasma coenzyme Q₁₀ concentration (µg/mL) and area under plasma coenzyme Q₁₀ concentration-time curve (AUC) of 1 - 24 hr after administration (µg/mL×hr) | | | | | | |
|---|---|---|---|---|---|---|
| | plasma coenzyme Q₁₀ concentration (hr) | | | | | AUC |
| Experiment No. | 1 | 2 | 4 | 8 | 24 | |
| (4-1) | 0.92 | 2.01 | 1.23 | 0.54 | 0.35 | 15.4 |
| (4-2) | 0.72 | 2.84 | 1.59 | 1.01 | 0.33 | 22.1 |
| (4-3) | 0.76 | 1.85 | 1.33 | 0.97 | 0.27 | 19.1 |
| (4-4) | 0.72 | 1.53 | 0.89 | 0.50 | 0.41 | 13.6 |
| (4-5) | 0.81 | 1.90 | 1.70 | 1.21 | 0.27 | 22.6 |
| (4-6) | 0.78 | 1.42 | 0.87 | 0.65 | 0.32 | 14.2 |
| (4-7) | 0.65 | 1.62 | 1.33 | 0.69 | 0.38 | 16.7 |
| (4-8) | 0.94 | 2.22 | 1.29 | 0.99 | 0.34 | 20.3 |
| (4-9) | 0.88 | 2.08 | 1.53 | 1.09 | 0.59 | 23.7 |
| (4-10) | 0.64 | 2.22 | 1.28 | 0.78 | 0.44 | 18.7 |
| (4-11) | 0.81 | 2.23 | 1.61 | 1.42 | 0.34 | 25.5 |

| | | | | | | |
|---|---|---|---|---|---|---|
| The data shows an average value of each n=5. | | | | | | |

From the above-mentioned results, in the case of a composition containing reduced coenzyme Q₁₀, lysolecithin, safflower oil (sufflower oil containing high oleic acid) and, as various surfactants, glycerol fatty acid ester or organic acid monoglyceride, it has been clarified that a composition containing organic acid monoglycerides such as acetic acid monoglyceride, citric acid monoglyceride and the like or glycerol fatty acid esters such as triglycerol monostearate, pentaglycerol trimyristate, decaglycerol monooleate, decaglycerol monostearate and tetraglycerol monolaurate is particularly superior in the oral absorbability.

### (Example 6)

### <Preparation of test substance administration solution>

Various fats and oils shown in Table 12 and soybean-derived lysolecithin (EMULTOP IP manufactured by Degussa) were mixed at a weight ratio 8:1 with heating to about 50°C. After mixing, reduced coenzyme Q₁₀ was added to various fats and oils/lysolecithin (weight ratio 8/1), and the mixture was dissolved by heating to about 60°C to give an administration solution (reduced coenzyme Q₁₀/lysolecithin weight ratio 1/5.5) having a reduced coenzyme Q₁₀ concentration of 2 wt%.

**(Table 12)**

| experiment No. | oil species | trade name | manufacturer |
|---|---|---|---|
| (5-1) | dietary safflower oil (oleic acid content in constituent fatty acid: not more than 30%) | Safflower oil fresh E | Sanwa Yushi Co., Ltd. |
| (5-2) | sufflower oil containing high oleic acid (oleic acid content in constituent fatty acid: 77%) | Ichibanshibori safflower oil | J-OIL MILLS INC. |
| (5-3) | olive oil(extra-virgin) | BOSCO EXTRA VIRGIN OLIVE OIL | BOSCO (Italy) |
| (5-4) | almond oil | almond oil | SIGMA |
| (5-5) | sesame oil | sesame oil | Yamakei Sangyo co., Ltd. |
| (5-6) | rice oil | rice oil | Tsuno Food Industrial Co., Ltd. |
| (5-7) | cottonseed oil | cottonseed oil | Yamakei Sangyo co., Ltd. |
| (5-8) | medium chain triglyceride (MCT) | Actor M-2 | RIKEN VITAMIN CO., LTD. |

### <Experiment system>

The above-mentioned test substance administration solution was orally administered to 11-week-old male Sprague-Dawley rats (supplier: Japan SLC, Inc.) at a dose of reduced coenzyme Q₁₀ of 30 mg/kg. The blood was taken from each rat at 1, 2, 4, 8 and 24 hr after test substance administration. The obtained blood was centrifuged to plasma. Thereafter, an oxidization treatment of plasma reduced coenzyme Q₁₀ and an extraction treatment of oxidized coenzyme Q₁₀ were performed and plasma coenzyme Q₁₀ concentration was measured as oxidized coenzyme Q₁₀ using HPLC. The plasma coenzyme Q₁₀ concentration before administration was data processed as 0 µg/ml.

### <Results>

The changes in the plasma coenzyme Q₁₀ concentration and areas under plasma coenzyme Q₁₀ concentration-time curve (AUC) of 0 hr - 24 hr when the above-mentioned test substance administration solution was orally administered are shown in Table 13.

**(Table 13)**

| time course changes of plasma coenzyme Q₁₀ concentration (µg/mL) and area under plasma coenzyme Q₁₀ concentration-time curve (AUC) of 0 - 24 hr after administration (µg/mL×hr) | | | | | | |
|---|---|---|---|---|---|---|
| | plasma coenzyme Q₁₀ concentration (hr) | | | | | AUC |
| Experiment No. | 1 | 2 | 4 | 8 | 24 | |
| (5-1) | 0.60 | 2.56 | 1.31 | 0.59 | 0.37 | 17.3 |
| (5-2) | 0.67 | 3.24 | 1.67 | 0.73 | 0.34 | 20.6 |
| (5-3) | 0.75 | 2.46 | 1.56 | 0.72 | 0.23 | 18.2 |
| (5-4) | 0.87 | 3.32 | 1.59 | 0.53 | 0.23 | 17.7 |
| (5-5) | 1.18 | 1.65 | 1.26 | 0.67 | 0.31 | 16.7 |
| (5-6) | 0.72 | 2.37 | 1.17 | 0.43 | 0.30 | 14.5 |
| (5-7) | 1.15 | 3.14 | 1.87 | 0.74 | 0.42 | 22.2 |
| (5-8) | 0.31 | 1.01 | 1.17 | 0.57 | 0.28 | 13.3 |

| | | | | | | |
|---|---|---|---|---|---|---|
| The data shows an average value of each n=5. | | | | | | |

From the above-mentioned results, with regard to the composition containing reduced coenzyme Q₁₀ and lysolecithin of the present invention, it has been clarified that particularly superior oral absorbability is afforded when safflower oil, sufflower oil containing high oleic acid, olive oil, almond oil, rice oil or cottonseed oil is used as fat and oil, and that a combined use of, from the same safflower oils, fat and oil having high oleic acid content in constituent fatty acid affords more superior oral absorbability.

### (Example 7)

### <Preparation of test substance administration solution>

Safflower oil (safflower oil containing high oleic acid, oleic acid content in constituent fatty acid: 77%), various polyglycerol fatty acid esters shown in Table 14 and soybean-derived lysolecithin (EMULTOP IP manufactured by Degussa) were mixed at a weight ratio 7:1:1 with heating to about 50°C. After mixing, reduced coenzyme Q₁₀ was added to safflower oil/polyglycerol fatty acid ester/lysolecithin (weight ratio 7/1/1), and the mixture was dissolved by heating to about 60°C to give an administration solution (reduced coenzyme Q₁₀/lysolecithin weight ratio 1/5.5) having a reduced coenzyme Q₁₀ (described in Production Example 1) concentration of 2 wt%.

**(Table 14)**

| Experiment No. | polyglycerol fatty acid ester species | trade name | manufacturer | HLB |
|---|---|---|---|---|
| (6-1) | tetraglycerol monolaurate | SY-Glyster ML-310 | Sakamoto Yakuhin Kogyo Co., Ltd. | 10.3 |
| (6-2) | hexaglycerol monooleate | SUNSOFT Q-17F | Taiyo Kagaku Co., Ltd. | 10.5 |
| (6-3) | decaglycerol monooleate | SY-Glyster MO-3S | Sakamoto Yakuhin Kogyo Co., Ltd. | 8.8 |

### <Experiment system>

The above-mentioned test substance administration solution was orally administered to 9-week-old male Sprague-Dawley rats (supplier: Japan SLC, Inc.) at a dose of reduced coenzyme Q₁₀ of 30 mg/kg. The blood was taken from each rat at 1, 2, 4, 8 and 24 hr after test substance administration. The obtained blood was centrifuged to plasma. Thereafter, an oxidization treatment of plasma reduced coenzyme Q₁₀ and an extraction treatment of oxidized coenzyme Q₁₀ were performed and plasma coenzyme Q₁₀ concentration was measured as oxidized coenzyme Q₁₀ using HPLC.

### <Results>

The changes in the plasma coenzyme Q₁₀ concentration and areas under plasma coenzyme Q₁₀ concentration-time curve (AUC) of 1 hr - 24 hr when the above-mentioned test substance administration solution was orally administered are shown in Table 15.

**(Table 15)**

| time course changes of plasma coenzyme Q₁₀ concentration (µg/mL) and area under plasma coenzyme Q₁₀ concentration-time curve (AUC) of 1 - 24 hr after administration (µg/mL×hr) | | | | | | |
|---|---|---|---|---|---|---|
| | Plasma coenzyme Q₁₀ concentration (hr) | | | | | |
| Experiment No. | 1 | 2 | 4 | 8 | 24 | AUC |
| (6-1) | 0.94 | 1.94 | 1.92 | 1.51 | 0.36 | 27.1 |
| (6-2) | 1.26 | 2.85 | 1.92 | 1.45 | 0.38 | 28.2 |
| (6-3) | 1.03 | 3.52 | 1.65 | 1.24 | 0.53 | 27.3 |

| | | | | | | |
|---|---|---|---|---|---|---|
| The data shows an average value of each n=5. | | | | | | |

From the above-mentioned results, in the case of a composition containing reduced coenzyme Q₁₀, lysolecithin, safflower oil (sufflower oil containing high oleic acid) and polyglycerol fatty acid ester, a higher maximum plasma coenzyme Q₁₀ concentration was often achieved when polyglycerol fatty acid ester showing high degree of polymerization of glycerol was used as polyglycerol fatty acid ester.

### (Example 8)

### <Preparation of test substance administration solution>

Safflower oil (safflower oil containing high oleic acid, oleic acid content in constituent fatty acid : 77%), various surfactants shown in Table 16 and soybean-derived lysolecithin (EMULTOP IP manufactured by Degussa or SLP-White Lyso manufactured by Tsuji Oil Mill Co., Ltd.) were mixed at a weight ratio 7:1:1 with heating to about 50°C. After mixing, reduced coenzyme Q₁₀ was added to safflower oil/surfactant/lysolecithin (weight ratio 7:1:1), and the mixture was dissolved by heating to about 60°C to give an administration solution (reduced coenzyme Q₁₀/lysolecithin weight ratio 1/5.5) having a reduced coenzyme Q₁₀ (described in Production Example 1) concentration of 2 wt%.

**(Table 16)**

| Experiment No. | lysolecithin species | surfactant species | trade name | manufacturer | HLB of surfactant |
|---|---|---|---|---|---|
| (7-1) | SLP-White Lyso | citric acid monoglyceride | SUNSOFT No.623M | Taiyo Kagaku Co., Ltd. | 7 |
| (7-2) | Emultop IP | citric acid monoglyceride | SUNSOFT No.623M | Taiyo Kagaku Co., Ltd. | 7 |
| (7-3) | Emultop IP | diglycerol monooleate | poem DO-100V | RIKEN VITAMIN CO., LTD. | 8 |
| (7-4) | Emultop IP | monoglycerol linoleate | Emulsy MU | RIKEN VITAMIN CO., LTD. | 4.2 |
| (7-5) | Emultop IP | monoglycerol caprylate | poem M-200 | RIKEN VITAMIN CO., LTD. | 6.8 |
| (7-6) | Emultop IP | monoglycerol stearate | MGS-F20V | Nihon Surfactant Co., Ltd. | 7.0 |
| (7-7) | Emultop IP | diacetyltartaric acid monoglyceride | poem W-70 | RIKEN VITAMIN CO., LTD. | 9.5 |
| (7-8) | Emultop IP | monoglycerol oleate | Excel O-95R | Kao Corporation | 3.5 |
| (7-9) | Emultop IP | sorbitan polyoxyethylene monooleate | Tween80 | MP Biomedicals, LLC. | 15 |
| (7-10) | Emultop IP | sorbitan monooleate | Span80 | Wako Pure Chemical Industries, Ltd. | 4.3 |

### <Experiment system>

The above-mentioned test substance administration solution was orally administered to 11-week-old male Sprague-Dawley rats (supplier: Japan SLC, Inc.) at a dose of reduced coenzyme Q₁₀ of 30 mg/kg. The blood was taken from each rat at 1, 2, 4, 8 and 24 hr after test substance administration. The obtained blood was centrifuged to plasma. Thereafter, an oxidization treatment of plasma reduced coenzyme Q₁₀ and an extraction treatment of oxidized coenzyme Q₁₀ were performed and plasma coenzyme Q₁₀ concentration was measured as oxidized coenzyme Q₁₀ using HPLC. The plasma coenzyme Q₁₀ concentration before administration was data processed as 0 µg/ml.

### <Results>

The changes in the plasma coenzyme Q₁₀ concentration and areas under plasma coenzyme Q₁₀ concentration-time curve (AUC) of 0 hr - 24 hr when the above-mentioned test substance administration solution was orally administered are shown in Table 17.

**(Table 17)**

| time course changes of plasma coenzyme Q₁₀ concentration (µg/mL) and area under plasma coenzyme Q₁₀ concentration-time curve (AUC) (µg/mL×hr) of 0 - 24 hr after administration | | | | | | |
|---|---|---|---|---|---|---|
| | plasma coenzyme Q₁₀ concentration (hr) | | | | | AUC |
| Experiment No. | 1 | 2 | 4 | 8 | 24 | |
| (7-1) | 1.31 | 3.87 | 2.49 | 1.13 | 0.80 | 32.3 |
| (7-2) | 1.08 | 2.87 | 1.62 | 0.61 | 0.53 | 20.6 |
| (7-3) | 0.74 | 2.79 | 1.60 | 0.73 | 0.47 | 20.7 |
| (7-4) | 1.33 | 3.21 | 1.82 | 1.26 | 0.41 | 27.5 |
| (7-5) | 0.97 | 2.13 | 1.97 | 1.42 | 0.34 | 26.9 |
| (7-6) | 1.09 | 4.30 | 2.47 | 0.70 | 0.42 | 25.4 |
| (7-7) | 1.08 | 3.43 | 2.05 | 1.21 | 0.71 | 30.1 |
| (7-8) | 1.26 | 2.78 | 2.03 | 0.62 | 0.38 | 20.8 |
| (7-9) | 0.39 | 2.56 | 1.40 | 0.69 | 0.27 | 17.4 |
| (7-10) | 0.45 | 2.64 | 2.32 | 0.58 | 0.42 | 20.5 |

| | | | | | | |
|---|---|---|---|---|---|---|
| The data shows an average value of each n=4. | | | | | | |

### (Example 9)

### <Preparation of test substance administration solution>

Safflower oil (safflower oil containing high oleic acid, oleic acid content in constituent fatty acid : 77%), and soybean-derived lysolecithin (EMULTOP IP manufactured by Degussa) were mixed to achieve the weight ratio shown in Table 18 with heating to about 50°C. After mixing, reduced coenzyme Q₁₀ was added to safflower oil/lysolecithin, and the mixture was dissolved by heating to about 60°C to give an administration solution having a reduced coenzyme Q₁₀ (described in Production Example 1) concentration of 10 wt%, which was sealed in a gelatin capsule at a dose of reduced coenzyme Q₁₀ of 10 mg/kg. As a comparison target, an administration solution having a reduced coenzyme Q₁₀ concentration of 10 wt% was prepared without using lysolecithin, adding reduced coenzyme Q₁₀ to MCT as a fat and oil component, similarly dissolving by heating and sealing same in a gelatin capsule.

### <Experiment system>

The above-mentioned test substance administration solution was orally administered to 13-week-old male Sprague-Dawley rats (supplier: Japan SLC, Inc.) at a dose of reduced coenzyme Q₁₀ of 10 mg/kg. The blood was taken from each rat at 1, 2, 4, 6, 8, 10 and 24 hr after test substance administration. The obtained blood was centrifuged to plasma. Thereafter, an oxidization treatment of plasma reduced coenzyme Q₁₀ and an extraction treatment of oxidized coenzyme Q₁₀ were performed and plasma coenzyme Q₁₀ concentration was measured as oxidized coenzyme Q₁₀ using HPLC. The plasma coenzyme Q₁₀ concentration before administration was data processed as 0 µg/ml.

### <Results>

The changes in the plasma coenzyme Q₁₀ concentration and areas under plasma coenzyme Q₁₀ concentration-time curve (AUC) of 0 hr - 24 hr when the above-mentioned test substance administration solution was orally administered are shown in Table 18.

**(Table 18)**

| time course changes of plasma coenzyme Q₁₀ concentration (µg/mL) and area under plasma coenzyme Q₁₀ concentration-time curve (AUC) (µg/mL×hr) of 0 - 24 hr after administration | | | | | | | | | | |
|---|---|---|---|---|---|---|---|---|---|---|
| Experiment No. | fat and oil used | reduced coenzyme Q₁₀ /fat and oil /lysolecithin weight ratio | plasma coenzyme Q₁₀ concentration (hr) | | | | | | | |
| | | | 1 | 2 | 4 | 6 | 8 | 10 | 24 | AUC |
| (8-1) | sufflower oil containing high oleic acid | 1/8.5/0.5 | 1.19 | 0.78 | 0.57 | 0.35 | - | 0.24 | 0.20 | 8.10 |
| (8-2) | sufflower oil containing high oleic acid | 1/8/1 | 1.91 | 1.46 | 1.00 | 0.38 | - | 0.23 | 0.26 | 11.1 |
| (8-3) | sufflower oil containing high oleic acid | 1/7.5/1.5 | 1.31 | 1.48 | 0.52 | 0.24 | - | 0.21 | 0.16 | 8.23 |
| (8-4) | MCT | 1/9/0 | 0.52 | 0.94 | 0.49 | - | 0.20 | - | 0.25 | 7.80 |

| | | | | | | | | | | |
|---|---|---|---|---|---|---|---|---|---|---|
| The data shows an average value of each n=4. | | | | | | | | | | |

From the above-mentioned results, it has been confirmed that the composition of the present invention containing 0.5-to 1.5-fold weight of lysolecithin relative to reduced coenzyme Q₁₀ is superior in the oral absorbability particularly based on the maximum plasma coenzyme Q₁₀ concentration by capsule administration as compared to non-use of lysolecithin. When the mixing ratio of lysolecithin and safflower oil (sufflower oil containing high oleic acid) was 1/8, that is, when the weight ratio of lysolecithin and reduced coenzyme Q₁₀ in the composition is 1/1, the oral absorbability tended to be particularly superior.

### (Example 10)

### <Preparation of test substance administration solution>

Safflower oil (safflower oil containing high oleic acid, oleic acid content in constituent fatty acid : 77%), various surfactants shown in Table 19 and soybean-derived lysolecithin (EMULTOP IP manufactured by Degussa) were mixed at a mixing ratio shown in Table 19 with heating to about 50°C. Reduced coenzyme Q₁₀ was added thereto, and the mixture was dissolved by heating to about 60°C to give an administration solution (reduced coenzyme Q₁₀/lysolecithin weight ratio 1/5.5) having a reduced coenzyme Q₁₀ (described in Production Example 1) concentration of 2 wt%. In addition, as a comparison target, an administration solution without lysolecithin and containing reduced coenzyme Q₁₀ dissolved in safflower oil (safflower oil containing high oleic acid, oleic acid content in constituent fatty acid: 77%) alone having a reduced coenzyme Q₁₀ concentration of 2 wt%, and one without lysolecithin and containing tetraglycerol monolaurate (SY-Glyster ML-310 manufactured by Sakamoto Yakuhin Kogyo Co., Ltd.) alone as a surfactant were prepared.

### <Experiment system>

12- to 16-week-old male Sprague-Dawley rats (supplier: Japan SLC, Inc.) were fasted from the evening of one day before the experiment and the above-mentioned test substance administration solution was orally administered at a dose of reduced coenzyme Q₁₀ of 30 mg/kg. The blood was taken from each rat at 1, 2, 4 and 8 hr after test substance administration. The obtained blood was centrifuged to plasma. Thereafter, an oxidization treatment of plasma reduced coenzyme Q₁₀ and an extraction treatment of oxidized coenzyme Q₁₀ were performed and plasma coenzyme Q₁₀ concentration was measured as oxidized coenzyme Q₁₀ using HPLC. The plasma coenzyme Q₁₀ concentration before administration was data processed as 0 µg/ml.

### <Results>

The changes in the plasma coenzyme Q₁₀ concentration and areas under plasma coenzyme Q₁₀ concentration-time curve (AUC) of 0 hr - 8 hr when the above-mentioned test substance administration solution was orally administered are shown in Table 19.

**(Table 19)**

| time course changes of plasma coenzyme Q₁₀ concentration (µg/mL) and area under plasma coenzyme Q₁₀ concentration-time curve (AUC) (µg/mL×hr) of 0 - 8 hr after administration | | | | | | | | |
|---|---|---|---|---|---|---|---|---|
| | | | lysolecithin/fat and oil/ surfactant ratio | plasma coenzyme Q₁₀ concentration (hr) | | | | AUC |
| Experiment No. | lysolecithin | surfactant species (trade name) | (weight ratio) | 1 | 2 | 4 | 8 | |
| (9-1) | Emultop IP | citric acid monoglyceride (SUNSOFT No.623M) | 1/7/1 | 0.11 | 1.36 | 1.49 | 0.23 | 7.07 |
| (9-2) | Emultop IP | none | 1/8/0 | 0.08 | 0.88 | 0.40 | 0.61 | 3.84 |
| (9-3) | none | none | fat and oil alone | 0.13 | 0.45 | 0.39 | 0.27 | 2.52 |
| (9-4) | Emultop IP | none | 1/8/0 | 0.23 | 0.83 | 0.83 | 0.25 | 4.49 |
| (9-5) | Emultop IP | monoglycerol linoleate (Emulsy MU) | 1/7/1 | 0.26 | 0.94 | 0.49 | 0.23 | 3.59 |
| (9-6) | Emultop IP | monoglycerol stearate (MGS-F20V) | 1/7.5/0.5 | 0.20 | 1.26 | 0.63 | 0.27 | 4.53 |
| (9-7) | Emultop IP | monoglycerol caprylate (poem M-200) | 1/7/1 | 0.34 | 1.54 | 0.64 | 0.38 | 5.32 |
| (9-8) | Emultop IP | diacetyltartaric acid monoglyceride (poem W-70) | 1/7/1 | 0.16 | 1.03 | 0.36 | 0.47 | 3.74 |
| (9-9) | none | tetraglycerol monolaurate (SY-Glyster ML-310) | 0/8/1 | 0.14 | 0.48 | 0.41 | 0.23 | 2.54 |

Generally, liposoluble substances such as coenzyme Q₁₀ are absorbed more by ingestion with food but hardly absorbed during fasting. From the above-mentioned results, it has been clarified that the composition of the present invention containing reduced coenzyme Q₁₀, lysolecithin and safflower oil (sufflower oil containing high oleic acid) shows superior absorbability during fasting, namely, in hungry rats, and that a combined use of a surfactant often results in more superior oral absorbability.

### (Example 11)

1 g of reduced coenzyme Q₁₀ obtained in Production Example 1 was added to a mixture (9 g) of medium chain triglyceride (MCT) and a surfactant described in Table 20 (weight ratio of MCT and surfactant 8/1), and the mixture was preserved at 40°C in the air. For comparison, a composition containing 1 g of reduced coenzyme Q₁₀ added to 9 g of MCT was similarly preserved. The weight ratio of reduced coenzyme Q₁₀/oxidized coenzyme Q₁₀ after lapse of 7 days is shown in Table 20.

**(Table 20)**

| | |
|---|---|
| surfactant | R |
| lysolecithin | 96.3/3.7 |
| Tween80 | 88.5/11.5 |
| none | 96.4/3.6 |

| | |
|---|---|
| R: weight ratio of reduced coenzyme Q₁₀/oxidized coenzyme Q₁₀ | |

Conventionally, it has been known that reduced coenzyme Q₁₀ shows degraded oxidization stability depending on the kind of the surfactant used. From the above-mentioned results, it has been clarified that composition containing reduced coenzyme Q₁₀ and lysolecithin of the present invention does not show degraded oxidization stability of reduced coenzyme Q₁₀.

### (Formulation Example 1: soft capsule)

To a mixture of medium chain triglyceride (MCT), lysolecithin (EMULTOP IP manufactured by Degussa) and beeswax was added at 40°C the crystal (reduced coenzyme Q₁₀) obtained in Production Example 1. The obtained mixture was processed to give a gelatin soft capsule preparation containing the following ingredients by a conventional method.

| | | |
|---|---|---|
| reduced coenzyme Q₁₀ | 10 | parts by weight |
| lysolecithin | 10 | parts by weight |
| beeswax | 5 | parts by weight |
| medium chain triglyceride | 75 | parts by weight |

### (Formulation Example 2: hard capsule)

Reduced coenzyme Q₁₀ obtained in Production Example 1, lysolecithin (EMULTOP IP manufactured by Degussa), crystalline cellulose (Avicel), cornstarch and lactose were mixed, and further mixed with magnesium stearate to give a mixed powder. The obtained mixed powder was filled in a hard capsule by a conventional method to give a hard capsule preparation containing the following ingredients.

| | | |
|---|---|---|
| reduced coenzyme Q₁₀ | 15 | parts by weight |
| lysolecithin | 10 | parts by weight |
| crystalline cellulose (Avicel) | 25 | parts by weight |
| cornstarch | 10 | parts by weight |
| lactose | 39 | parts by weight |
| magnesium stearate | 1 | part by weight |

### (Formulation Example 3: tablet)

Reduced coenzyme Q₁₀ obtained in Production Example 1, lysolecithin (EMULTOP IP manufactured by Degussa), crystalline cellulose (Avicel), cornstarch and lactose were mixed, and further mixed with magnesium stearate to give a mixed powder. The obtained mixed powder was processed by a conventional method to give a tablet containing the following ingredients.

| | | |
|---|---|---|
| reduced coenzyme Q₁₀ | 15 | parts by weight |
| lysolecithin | 15 | parts by weight |
| crystalline cellulose (Avicel) | 20 | parts by weight |
| cornstarch | 15 | parts by weight |
| lactose | 34 | parts by weight |
| magnesium stearate | 1 | part by weight |

### (Formulation Example 4: chewable tablet)

Reduced coenzyme Q₁₀ obtained in Production Example 1, lysolecithin (EMULTOP IP manufactured by Degussa), crystalline cellulose, cornstarch and lactose were mixed, and further mixed with magnesium stearate to give a mixed powder. The obtained mixed powder was processed by a conventional method to give a tablet containing the following ingredients.

| | | |
|---|---|---|
| reduced coenzyme Q₁₀ | 15 | parts by weight |
| lysolecithin | 5 | parts by weight |
| crystalline cellulose (Avicel) | 10 | parts by weight |
| cornstarch | 5 | parts by weight |
| lactose | 64 | parts by weight |
| magnesium stearate | 1 | part by weight |

While some of the embodiments of the present invention have been described in detail in the above, it is, however, possible for those of ordinary skill in the art to make various modifications and changes to the particular embodiments shown without substantially departing from the teaching and advantages of the present invention. Such modifications and changes are encompassed in the spirit and scope of the present invention as set forth in the appended claims.

## Claims

1. A composition comprising reduced coenzyme Q₁₀ and lysolecithin wherein the weight ratio of reduced coenzyme Q₁₀ and lysolecithin is not less than 10/90 and not more than 90/10.

2. The composition of claim 1, wherein the content of reduced coenzyme Q₁₀ in the composition is not less than 0.1 wt% of the total weight of the composition.

3. The composition of claim 1 or 2, wherein the content of lysolecithin in the composition is not less than 0.1 wt% of the total weight of the composition.

4. The composition of any one of claims 1 to 3, further comprising at least one kind selected from the group consisting of an oil and fat, a surfactant, a higher fatty acid, ethanol and water.

5. The composition of claim 4, wherein the oil and fat is at least one kind selected from the group consisting of safflower oil, olive oil, almond oil, rice oil, rapeseed oil and cottonseed oil.

6. The composition of claim 4, wherein the oil and fat is an oil and fat wherein oleic acid accounts for not less than 50% of the constituent fatty acids thereof.

7. The composition of claim 4, wherein the surfactant is at least one kind of a glycerol fatty acid ester and an organic acid monoglyceride.

8. The composition of claim 7, wherein the glycerol fatty acid ester is at least one kind of surfactant selected from the group consisting of triglycerol monostearate, pentaglycerol trimyristate, decaglycerol monooleate, decaglycerol monostearate, tetraglycerol monolaurate, hexaglycerol monooleate, monoglycerol caprylate, monoglycerol linolate, and monoglycerol stearate.

9. The composition of claim 4, wherein the higher fatty acid is oleic acid.

10. The composition of any one of claims 4 to 9, wherein the composition is liquid or slurry.

11. The composition of claim 10, wherein the total amount of oil and fat, surfactant, higher fatty acid, ethanol and water to the total weight of the composition is not less than 20 wt%.

12. A tablet, powder, chewable tablet, pill, or capsule obtained by processing the composition of any one of claims 1 to 11.

13. The capsule of claim 12, which is a soft capsule.

14. A method of increasing the absorbability of reduced coenzyme Q₁₀ comprising preparing a composition wherein reduced coenzyme Q₁₀ is ingested in the co-presence of lysolecithin.

## Patentansprüche

1. Zusammensetzung, die reduziertes Coenzym Q₁₀ und Lysolecithin umfasst, wobei das Gewichtsverhältnis von reduziertem Coenzym Q₁₀ und Lysolecithin nicht weniger als 10/90 und nicht mehr als 90/10 beträgt.

2. Zusammensetzung nach Anspruch 1, wobei der Gehalt des reduzierten Coenzyms Q₁₀ in der Zusammensetzung nicht weniger als 0,1 Gew.% des Gesamtgewichts der Zusammensetzung ist.

3. Zusammensetzung nach Anspruch 1 oder 2, wobei der Gehalt des Lysolecithins in der Zusammensetzung nicht weniger als 0,1 Gew.% des Gesamtgewichts der Zusammensetzung ist.

4. Zusammensetzung nach mindestens einem der Ansprüche 1 bis 3, die ferner mindestens eine Komponente umfasst, die aus der Gruppe ausgewählt ist, die aus einem Öl und Fett, einem Tensid, einer höheren Fettsäure, Ethanol und Wasser besteht.

5. Zusammensetzung nach Anspruch 4, wobei das Öl und Fett mindestens eines ist, das aus der Gruppe ausgewählt ist, die aus Distelöl, Olivenöl, Mandelöl, Reisöl, Rapsöl und Baumwollsamenöl besteht.

6. Zusammensetzung nach Anspruch 4, wobei das Öl und Fett ein Öl und Fett sind, in denen der Anteil der Ölsäure nicht weniger als 50% der Fettsäurebestandteile davon ausmacht.

7. Zusammensetzung nach Anspruch 4, in der das Tensid mindestens eines von einem Glycerinfettsäureester und einem Monoglycerid einer organischen Säure ist.

8. Zusammensetzung nach Anspruch 7, in der der Glycerinfettsäureester mindestens eine Art von Tensid ist, die aus der Gruppe ausgewählt ist, die aus Triglycerinmonostearat, Pentaglycerintrimyristat, Decaglycerinmonooleat, Decaglycerinmonostearat, Tetraglycerinmonolaurat, Hexaglycerinmonooleat, Monoglycerincaprylat, Monoglycerinlinolat und Monoglycerinstearat besteht.

9. Zusammensetzung nach Anspruch 4, in der die höhere Fettsäure Ölsäure ist.

10. Zusammensetzung nach mindestens einem der Ansprüche 4 bis 9, wobei die Zusammensetzung eine Flüssigkeit oder eine Aufschlämmung ist.

11. Zusammensetzung nach Anspruch 10, in der die Gesamtmenge von Öl und Fett, Tensid, höherer Fettsäure, Ethanol und Wasser in Bezug auf das Gesamtgewicht der Zusammensetzung nicht weniger als 20 Gew.% beträgt.

12. Tablette, Pulver, kaubare Tablette, Pille oder Kapsel, die durch Verarbeitung der Zusammensetzung nach mindestens einem der Ansprüche 1 bis 11 erhalten ist.

13. Kapsel nach Anspruch 12, die eine Weichkapsel ist.

14. Verfahren zur Erhöhung der Absorptionsfähigkeit von reduziertem Coenzym Q₁₀, das die Herstellung einer Zusammensetzung umfasst, bei dem reduziertes Coenzym Q₁₀ in Gegenwart von Lysolecithin eingenommen wird.

## Revendications

1. Composition comprenant un coenzyme Q₁₀ réduit et de la lysolécithine dans laquelle le rapport massique de coenzyme Q₁₀ réduit et de lysolécithine n'est pas inférieur à 10/90 et n'est pas supérieur à 90/10.

2. Composition selon la revendication 1, dans laquelle la teneur de coenzyme Q₁₀ réduit dans la composition n'est pas inférieure à 0,1 % en poids du poids total de la composition.

3. Composition selon la revendication 1 ou 2, dans laquelle la teneur de lysolécithine dans la composition n'est pas inférieure à 0,1 % en poids du poids total de la composition.

4. Composition selon l'une quelconque des revendications 1 à 3, comprenant de plus au moins un type choisi dans le groupe constitué d'une huile et d'une graisse, d'un tensioactif, d'un acide gras supérieur, d'éthanol et d'eau.

5. Composition selon la revendication 4, dans laquelle l'huile et la graisse sont au moins un type choisi dans le groupe constitué d'huile de carthame, d'huile d'olive, d'huile d'amande, d'huile de riz, d'huile de tournesol et d'huile de coton.

6. Composition selon la revendication 4, dans laquelle l'huile et la graisse sont une huile et une graisse dans lesquelles l'acide oléique compte pour au moins 50 % des constituants d'acides gras de ceux-ci.

7. Composition selon la revendication 4, dans laquelle le tensioactif est au moins un type d'ester d'acide gras de glycérol et d'un monoglycéride d'acide organique.

8. Composition selon la revendication 7, dans laquelle l'ester d'acide gras de glycérol est au moins un type de tensioactif choisi dans le groupe constitué de monostéarate de triglycérol, de trimyristate de penta-glycérol, de monooléate de décaglycérol, de monostéarate de décaglycérol, de monolaurate de tétraglycérol, de monooléate d'hexaglycérol, de caprylate de monoglycérol, de linolate de monoglycérol et de stéarate de monoglycérol.

9. Composition selon la revendication 4, dans laquelle l'acide gras supérieur est l'acide oléique.

10. Composition selon l'une quelconque des revendications 4 à 9, dans laquelle la composition est liquide ou une suspension.

11. Composition selon la revendication 10, dans laquelle la quantité totale d'huile et de graisse, de tensioactif, d'acide gras supérieur, d'éthanol et d'eau par rapport au poids total de la composition n'est pas inférieure à 20 % en poids.

12. Comprimé, poudre, comprimé à mastiquer, pilule ou capsule obtenue par traitement de la composition selon l'une quelconque des revendications 1 à 11.

13. Capsule selon la revendication 12, laquelle est une capsule molle.

14. Procédé d'augmentation de la capacité d'absorption de coenzyme Q₁₀ réduit comprenant la préparation d'une composition dans lequel le coenzyme Q₁₀ réduit est ingéré en co-présence de lysolécithine.
